# EUROPEAN PATENT APPLICATION

(11) **EP 4 708 732 A1**
(43) Date of publication of application: **11.03.2026**
(21) Application number: 25200738.0
(22) Date of filing: 08.09.2025
(51) Int. Cl.: H04B 10/80, A61B 6/00

(54) **TRANSMISSION SYSTEMS OF MEDICAL DEVICES AND MEDICAL DEVICES**

(30) Priority: 06.09.2024 CN 202411253929; 19.05.2025 CN 202510645250; 15.08.2025 CN 202511150052
(71) Applicant: Shanghai United Imaging Healthcare Co., Ltd., Shanghai 201807 (CN)
(72) Inventor: ZHOU, Zerun, Shanghai, 201807 (CN); WANG, Dongming, Shanghai, 201807 (CN); ZHOU, Xiaofeng, Shanghai, 201807 (CN); SUN, Fengjie, Shanghai, 201807 (CN); WEN, Ruijian, Shanghai, 201807 (CN); SHI, Wei, Shanghai, 201807 (CN); ZHANG, Wei, Shanghai, 201807 (CN); YANG, Yansi, Shanghai, 201807 (CN); CHEN, Beibei, Shanghai, 201807 (CN); LV, Tong, Shanghai, 201807 (CN); HOU, Fangyan, Shanghai, 201807 (CN); WU, Yingdong, Shanghai, 201807 (CN); ZHANG, Wei, Shanghai, 201807 (CN); WANG, Hualing, Shanghai, 201807 (CN); SONG, Renjie, Shanghai, 201807 (CN); ZHANG, Yang, Shanghai, 201807 (CN)
(74) Representative: Wang, Bo

(57) **Abstract**

Embodiments of the present disclosure provide a transmission system of a medical device, comprising a first transceiver on a rotor and a second transceiver on a stator, wherein the first transceiver on the rotor is configured to transmit scanning data to and receive control signals from the second transceiver on the stator; the second transceiver on the stator is configured to transmit the control signals to and receive the scanning data from the first transceiver on the rotor; and a bidirectional transmission between the first transceiver and the second transceiver is wireless.

## Description

### CROSS-REFERENCE TO RELATED APPLICATIONS

This application claims priority to Chinese application No. 202411253929.5, filed on September 06, 2024, Chinese application No. 202510645250.9, filed on May 19, 2025, and Chinese application No. 2025111500521, filed on August 15, 2025, the entire contents of each of which are incorporated herein by reference.

### TECHNICAL FIELD

This disclosure relates to the field of medical devices, and in particular relates to a transmission system for a medical device.

### BACKGROUND

In rotary medical devices (e.g., Computerized Tomography (CT) devices, Radiation Therapy (RT) devices, etc.), a tube and a detector are arranged relative to each other on a rotor of a rotary medical device. During the rotation process, the rotor transmits scanning data collected by the detector to a stator in real time, so as to perform an image reconstruction. The stator transmits control signals (e.g., scanning instructions, control parameters, etc.) that control the tube and the detector to the rotor to control a scanning process. Therefore, the rotor receives the scanning instructions and obtains the scanning data, while the stator emits the scanning instructions and receives the scanning data. A bidirectional data transmission between the stator and the rotor is performed. In existing rotary medical devices, data always transmitted via cables on the rotor and the stator. However, cables occupy large space inside the rotary medical devices and are inconvenient for integration.

Therefore, it is desired to provide a transmission system for a medical device to solve the above problem.

### SUMMARY

One or more embodiments of the present disclosure provide a transmission system of a medical device, comprising a first transceiver on a rotor and a second transceiver on a stator. The first transceiver on the rotor is configured to transmit scanning data to and receive control signals from the second transceiver on the stator. The second transceiver on the stator is configured to transmit the control signals to and receive the scanning data from the first transceiver on the rotor. A bidirectional transmission between the first transceiver and the second transceiver is wireless.

In some embodiments, the bidirectional transmission between the first transceiver and the second transceiver is a transmission via wave-based signals.

In some embodiments, the bidirectional transmission between the first transceiver and the second transceiver is the transmission via optical waves, and a transmission system further includes a beam expander; the first transceiver or the second transceiver is configured to emit a first optical beam carrying data; the beam expander is configured to divide the first optical beam into a plurality of second optical beams, each of the plurality of second optical beams records at least part same data with the first optical beam; and the second transceiver or the first transceiver is configured to receive at least part of the plurality of second optical beams.

In some embodiments, the beam expander includes a wavelength division demultiplexer and a filter. The wavelength division demultiplexer is configured to divide the first optical beam into a plurality of second optical beams of a plurality of wavelengths, and the filter is configured to filter the plurality of second optical beams.

In some embodiments, the filter is a ring-shaped filter.

In some embodiments, the transmission system further includes an angle determination module. The angle determination module is configured to: determine an angle covered by an expanded optical spot; and determine, based on the angle, a first transceiver count or a second transceiver count. An interval angle between two adjacent first transceivers or an interval angle between two adjacent second transceivers.

In some embodiments, the transmission system includes a plurality of first transceivers and a second transceiver. The plurality of first transceivers are arranged at interval along the circumference of the rotor based on the interval angle.

In some embodiments, the transmission system includes a first transceiver and a plurality of second transceivers, the plurality of second transceivers are arranged outside the circumference of the rotor at interval based on the interval angle.

In some embodiments, the bidirectional transmission between the first transceiver and the second transceiver is a transmission via optical waves. The first transceiver is located at a circumference of the rotor, the stator is located at a rotational axis of the rotor. The transmission system further includes an optical path adjustment module between the first transceiver and the second transceiver. The optical path adjustment module is configured to adjust an optical direction of an optical beam emitted by the first transceiver or the second transceiver.

In some embodiments, the optical path adjustment module includes at least one adjustment unit located adjacent to the first transceiver of the second transceiver that serves as a receiver.

In some embodiments, the optical path adjustment module includes a first adjustment unit located adjacent to the first transceiver of the second transceiver that serves as a receiver and a second adjustment unit located adjacent to the first transceiver of the second transceiver that serves as a transmitter.

In some embodiments, the optical path adjustment module includes a direction-adjusting element selected from: an optical wedge, a reflector, or a diffractive grating.

In some embodiments, the transmission system further includes a controller configured to control the optical path adjustment module.

In some embodiments, the transmission system further includes a power detector configured to detect a power of an optical beam received by the first transceiver or the second transceiver, and transmit the power to the controller. The controller controls the optical path adjustment module based on the power.

In some embodiments, the transmission system further includes a vibration detector configured to detect vibration information of the rotor and transmit the vibration information to the controller. The controller controls the optical path adjustment module based on the vibration information.

In some embodiments, the bidirectional transmission between the first transceiver and the second transceiver is the transmission via electromagnetic waves. The first transceiver is located at a circumference of the rotor. The first transceiver includes a first phased-array antenna. The second transceiver includes a second phased-array antenna.

In some embodiments, the second phased-array antenna is located within a coverage area of radio frequency beams emitted from the first phased-array antenna.

In some embodiments, the transmission system further includes a synchronization module configured to obtain real-time motion parameters of the rotor. A beam controller configured to adjust the radio frequency beams emitted from the first phased-array antenna or the second phased-array antenna based on the motion parameters of the rotor and a pre-stored mapping relationship between motion parameters and beam parameters.

In some embodiments, the pre-stored mapping relationship between motion parameters and beam parameters is obtained by: dividing a circle that the rotor rotates into a plurality of angular regions; determining a beam parameter corresponding to each of the plurality of angular regions, based on the beam parameter corresponding to the angular region, a signal quality received by the second phased-array antenna satisfies a preset condition; generating the pre-stored mapping relationship between the motion parameters and the beam parameters. Each of the plurality of angular regions represents an angular area where the first phased-array antenna of the rotor possibly located during a rotation process with the rotor.

In some embodiments, the first phased-array antenna or the second phased-array antenna includes a plurality of phased-array elements, and the plurality of phased-array elements work at different frequencies or at different time interval.

One or more embodiments of the present disclosure provide a medical device comprising the transmission system.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering denotes the same structure, where:
FIG. 1 is a schematic diagram of an application scenario of a transmission system of a medical device according to some embodiments of the present disclosure;
FIG. 2 is a diagram of exemplary modules of a transmission system of a medical device according to some embodiments of the present disclosure;
FIG. 3 is a schematic diagram of a transmission system of a medical device according to some embodiments of the present disclosure;
FIG. 4A is an exemplary schematic diagram of a transmission system of a medical device according to some other embodiments of the present disclosure;
FIG. 4B is a schematic diagram of an exemplary arrangement of second optical beams according to some embodiments of the present disclosure;
FIG. 5 is a schematic diagram of a transmission system of a medical device according to some embodiments of the present disclosure;
FIG. 6A is a schematic diagram of an exemplary arrangement of an optical power amplifier according to some embodiments of the present disclosure;
FIG. 6B is a schematic diagram of an exemplary arrangement of an optical power amplifier according to some other embodiments of the present disclosure.
FIG. 7A is a schematic diagram of an exemplary arrangement of a closed-loop control module according to some embodiments of the present disclosure;
FIG. 7B is a schematic diagram of an exemplary arrangement of an angle determination module according to some other embodiments of the present disclosure;
FIG. 8A is a schematic diagram of an exemplary arrangement of an angle determination module according to some embodiments of the present disclosure;
FIG. 8B is a schematic diagram of an exemplary arrangement of an angle determination module according to some other embodiments of the present disclosure;
FIG. 8C is a schematic diagram of an exemplary arrangement of an angle determination module according to some other embodiments of the present disclosure;
FIG. 8D is a schematic diagram of a transmission system of a medical device according to some other embodiments of the present disclosure;
FIG. 9 is a schematic diagram of a transmission system of a medical device according to some other embodiments of the present disclosure;
FIG. 10A is a schematic diagram of an exemplary arrangement of at least one adjustment unit according to some embodiments of the present disclosure;
FIG. 10B is a schematic diagram of an exemplary arrangement of at least one adjustment unit according to some other embodiments of the present disclosure;
FIG. 11A is a schematic diagram of an exemplary arrangement of an adjustment unit according to some embodiments of the present disclosure
FIG. 11B is a schematic diagram of an exemplary arrangement of an adjustment unit according to some other embodiments of the present disclosure;
FIG. 12A is a schematic diagram of data transmission based on optical waves according to some embodiments of the present disclosure;
FIG. 12B is a schematic diagram of data transmission based on terahertz according to some embodiments of the present disclosure;
FIG. 12C is a schematic diagram of data transmission based on millimeter waves according to some embodiments of the present disclosure;
FIG. 13 is a schematic diagram of a transmission system of a medical device according to some other embodiments of the present disclosure;
FIG. 14 is a schematic diagram of a transmission system of a medical device according to some other embodiment of the present disclosure;
FIG. 15 is a schematic diagram of a transmission system of a medical device according to some other embodiment of the present disclosure;
FIG. 16 is a schematic diagram of controlling an optical path adjustment module according to some embodiments of the present disclosure;
FIG. 17 is a schematic diagram of a transmission system of a medical device according to some other embodiments of the present disclosure;
FIG. 18 is a schematic diagram of phased-array elements according to some embodiments of the present disclosure;
FIG. 19 is a flow diagram of a data transmission process of a transmission system of a medical device according to some embodiments of the present disclosure;
FIG. 20 is a flow diagram for determining a pre-stored mapping relationship between motion parameters and beam parameters according to some embodiments of the present disclosure; and
FIG. 21 is a schematic diagram of angular regions according to some embodiments of the present disclosure.

### DETAILED DESCRIPTION

In order to more clearly illustrate the technical solutions of the embodiments of the present disclosure, the accompanying drawings required to be used in the description of the embodiments will be briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system," "device," "unit," and/or "module" as used herein are used as a way to distinguish between different components, elements, parts, sections or assemblies at different levels. However, the words may be replaced by other expressions if other words accomplish the same purpose.

As shown in the present disclosure and the claims, unless the context clearly suggests an exception, the words "a," "an," "one," and/or "the" do not refer specifically to the singular, but may also include the plural. Generally, the terms "including," and "comprising" suggest only the inclusion of clearly identified operations and elements. In general, the terms "including," and "comprising" only suggest the inclusion of explicitly identified operations and elements that do not constitute an exclusive list, and the method or apparatus may also include other operations or elements.

Flowcharts are used in this disclosure to illustrate operations performed by a system in accordance with embodiments of the present disclosure. It should be appreciated that the preceding or following operations are not necessarily performed in an exact sequence. Instead, operations may be processed in reverse order or simultaneously. Also, it is possible to add other operations to these processes, or to remove an operation or operations from these processes.

In the medical fields, there are numerous types of rotary medical device. For illustration purpose, a computed tomography (CT) device is described below as an example. The CT device includes an X-ray tube, a detector, a gantry, and a scanning bed. The X-ray tube and the detector are arranged on a rotor of the CT device. Some components of the CT device that are stationary act as a stator. The CT device utilizes the tube together with the detector to make a cross-section scan around a certain part of a target object. The detector collects attenuation signals passing through the target object in real-time, and through the data transmission between the rotor and the stator, the data collected by the detector is transmitted to the stator, so as to perform data processing and image reconstruction, thereby obtaining a CT image of the target object. The stator transmits control signals (e.g., scanning instructions, control parameters, etc.) that control the tube and the detector to the rotor to control the scanning process.

In the related technologies, the data transmission between the rotor and the stator is often performed by a slip ring and a carbon brush. For example, a slip ring is arranged on a circumference of the rotor, and a carbon brush is arranged at a preset position of the stator, a contact between the slip ring and the carbon brush realizes data transmission. However, there are high installation requirements between the rotor and the stator when the data transmission is carried out.

In the related technologies, cables are arranged on a circumference of the rotor, and couples with cables of the stator to transmit data, which has problems such as large space occupation and inconvenient integration.

In order to solve the above problems, some embodiments of the present disclosure provide a transmission system of a medical device for performing a bidirectional and wireless data transmission. The transmission system includes a first transceiver located at the rotor and a second transceiver located at the stator. The stator is flexibly located at an outer circumference of the rotor, a rotational axis, or other positions. The first transceiver and the second transceiver can realize bidirectional wireless propagation of data through optical waves and/or electromagnetic waves, which makes the system integration convenient, eliminates cable connection, effectively reduces the space occupation, and avoids the situation where the length of the cable restricts the data transmission distance.

The technical solutions disclosed herein are hereinafter described in detail in conjunction with the accompanying drawings.

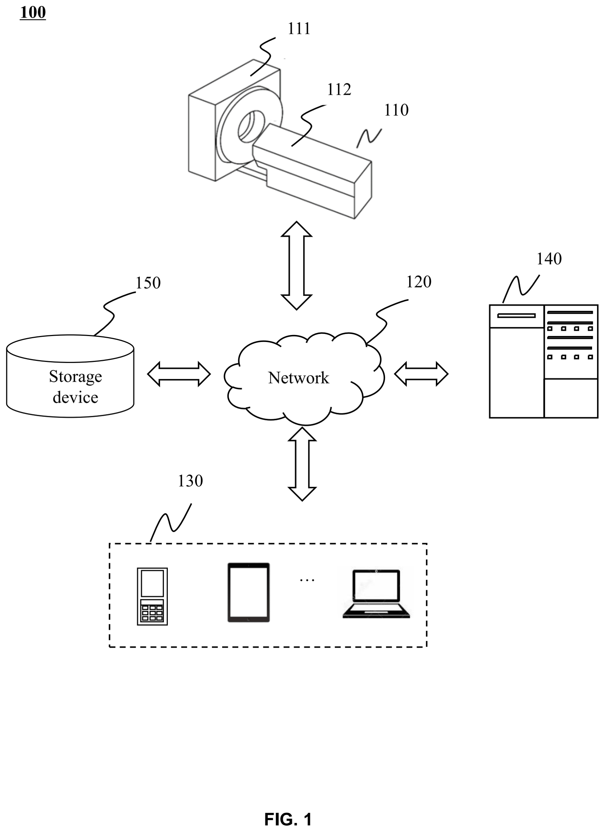

FIG. 1 is a schematic diagram of an application scenario of a transmission system of a medical device according to some embodiments of the present disclosure.

As shown in FIG. 1, an application scenario 100 of a transmission system for a medical device includes a medical device 110, a processor 120, a terminal device 130, a storage device 140, and a network 150.

The medical device 110 scans a target object in a detection area or a scanning area to obtain scanning data of the target object. In some embodiments, the medical device 110 may be a CT device, an RT device, etc. Take the CT device as an example, the CT device uses X-rays and computer processing technology to generate cross-sectional (tomographic) images of the target object with high resolution and tissue contrast, and has important applications in the fields of disease diagnosis and screening, trauma assessment, vascular imaging, and three-dimensional medical image reconstruction.

In some embodiments, the target object includes a biological object and/or a non-biological object. For example, the target object may include a patient, an artificial object, etc. In some embodiments, the target object includes a specific portion of a body, such as the head, the chest, the abdomen, etc., or any combination thereof. In some embodiments, the target object includes a specific organ, such as the heart, esophagus, trachea, bronchus, stomach, gallbladder, small intestine, colon, bladder, ureter, uterus, fallopian tube, or the like, or any combination thereof.

In some embodiments, the medical device 110 includes a gantry 111 and a scanning bed 112 as shown in FIG. 1.

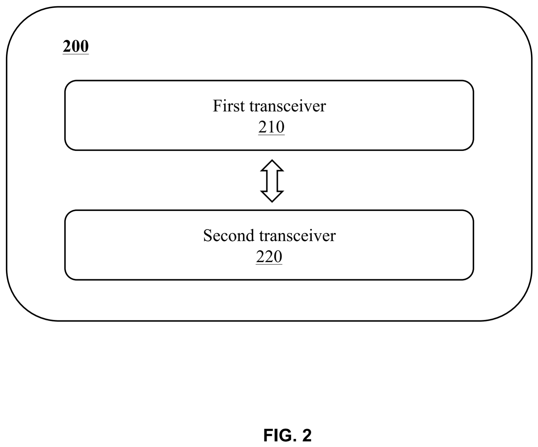

The gantry 111 is a main component of the medical device 110. The gantry 111 may provide physical support and holding space for scanning components (e.g., X-ray tubes, detectors, collimators, filters, rotary drive systems, a transmission system, etc.). In some embodiments, a transmission system 200 and the scanning components may be located in the gantry 111 of the medical device 110, or located in a vicinity of the medical device 110, as may be described in more detail in the relevant portion of the later section, such as FIG.2 to FIG.21.

The scanning bed 112 may provide support for a target object (e.g., a patient) to be scanned. For example, the target object may be placed on the scanning bed 112, moved into or out of an aperture of the gantry 111.

Network 120 may include any suitable network that facilitates the exchange of information and/or data for the application scenario 100. In some embodiments, one or more components of the application scenario 100 (e.g., the medical device 110, the terminal device 130, the processor 140, and the storage device 150) may transmit information and/or data to one or more other components of the application scenario 100 via the network 120. For example, the processor 140 may obtain the scanning data from the medical device 110 via the network 120, etc. In some embodiments, the network 120 may be any one or more of a wired network or a wireless network. In some embodiments, the network may have a plurality of topological structures, such as point-to-point, shared, centralized, or a combination of the plurality of topological structures.

The end device 130 may include a mobile device, a tablet, a laptop, etc., or any combination thereof. In some embodiments, the end device 130 may interact with other components in the application scenario 100 via the network 120. For example, the terminal device 130 may receive CT scanning data sent by the medical device 110, etc. In some embodiments, the terminal device 130 may receive information and/or instructions entered by a user (e.g., a user of the medical device 110, such as a physician, a medical technician, etc.) and send the received information and/or instructions to the medical device 110 or the processor 140 via the network 120. For example, the physician may enter instructions for operating the medical device 110 via the terminal device 130. In some embodiments, the terminal device 130 may display scanning results (e.g., generated CT images), etc. In some embodiments, the terminal device 130 may also be integrated with the medical device 110.

The processor 140 may process data and/or information obtained from the medical device 110, the terminal device 130, and/or the storage device 150. For example, the processor 140 may control the first transceiver or the second transceiver to emit a first optical beam, and the first optical beam may be an optical beam. For another example, the processor 140 may control a beam expander to divide the first optical beam emitted by the transmitter into a plurality of second optical beams of different wavelengths, and the transmitter may be one of the first transceiver or the second transceiver that sends the signal. For another example, the processor 140 may control the receiver to receive at least a portion of the plurality of second optical beams of different wavelengths, and the receiver may be one of the first transceiver or the second transceiver that receives the signal.

In some embodiments, the processor 140 may also be referred to as a controller for controlling an optical path adjustment module in the transmission system 200.

In some embodiments, the processor 140 may also be referred to as a processing module for adjusting radio frequency beams emitted by the first transceiver or the second transceiver.

In some embodiments, the processor 140 may be a single server or a group of servers. The group of servers may be centralized or distributed. In some embodiments, the processor 140 may be local or remote. The processor 140 may be directly connected to the medical device 110, the terminal device 130, and the storage device 150 to access stored or acquired information and/or data. In some embodiments, the processor 140 may be implemented on a cloud platform. Merely by way of example, the cloud platform may include a private cloud, a public cloud, a hybrid cloud, a community cloud, a distributed cloud, an on-premises cloud, a multi-tier cloud, or any combination thereof.

The storage device 150 may store data and/or instructions. In some embodiments, the storage device 150 may store data obtained from the medical device 110, the terminal device 130, and/or the processor 140. For example, the storage device 150 may store the scanning data acquired by the medical device 110. In some embodiments, the storage device 150 may store data and/or instructions used by the processor 140 to perform the exemplary manners described in the present disclosure. In some embodiments, the storage device 150 may include a mass storage device, a removable storage device, a volatile read-write memory, a read-only memory (ROM), or any combination thereof. In some embodiments, the storage device 150 may be implemented on a cloud platform.

In some embodiments, the storage device 150 may be connected to the network 120 to communicate with one or more components of the application scenario 100 (e.g., the medical device 110, the terminal device 130, the processor 140, etc.). One or more components of the application scenario 100 may access data or instructions stored in the storage device 150 via the network 120. In some embodiments, the storage device 150 may be directly connected to or in communication with one or more components of the application scenario 100. In some embodiments, the storage device 150 may be a part of the processor 140.

It should be noted that the foregoing description is located for illustrative purposes only and is not intended to limit the scope of the present disclosure. For a person of ordinary skill in the art, a wide variety of variations and modifications may be made under the guidance of the contents of the present disclosure. Features, structures, methods, and other characteristics of the exemplary embodiments described herein may be combined in various ways to obtain additional and/or alternative exemplary embodiments. However, these changes and modifications do not depart from the scope of the present disclosure.

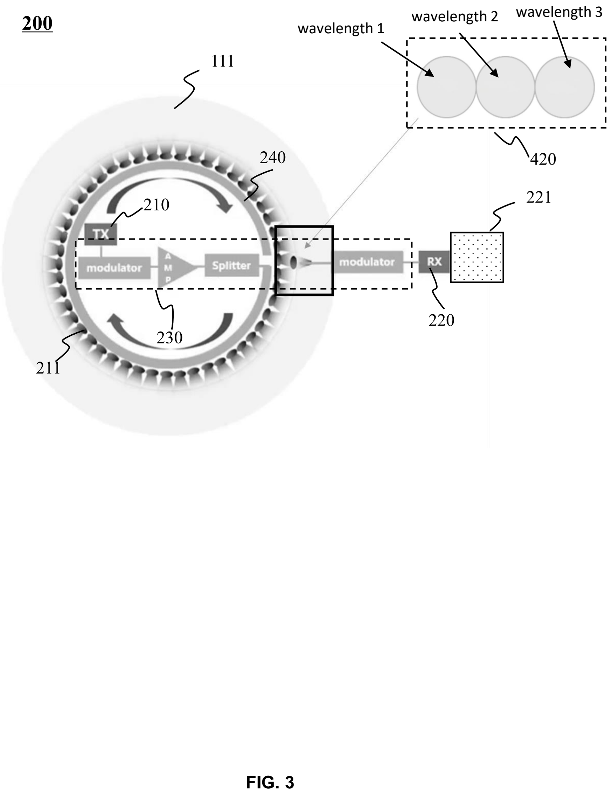

FIG. 2 is a diagram of exemplary modules of a transmission system of a medical device according to some embodiments of the present disclosure. FIG. 3 is a schematic diagram of a transmission system of a medical device according to some embodiments of the present disclosure. In some embodiments, the transmission system 200 may be part of the medical device 110 or integrated on the medical device 110. For example, when the medical device 110 is a CT device, the transmission system may be part of a slip ring (or a rotor) of the medical device 110 or mounted on the slip ring of the medical device 110.

As shown in FIG. 2, the transmission system 200 of the medical device (hereinafter referred to as the transmission system 200) includes a first transceiver 210 and a second transceiver 220. In some embodiments, as shown in FIG. 3, the first transceiver 210 is located at a rotor 211, and the second transceiver 220 is located at a stator 221 of the medical device.

The first transceiver 210 is configured to transmit scanning data to and receive control signals from the second transceiver on the stator 221. For example, the first transceiver 210 may act as a transmitter and transmit the scanning data acquired by the medical device to the second transceiver 220. As another example, the first transceiver 210 may act as a receiver and receive the control signals sent by the second transceiver 220.

In some embodiments, the first transceiver 210 or the second transceiver 220 is an optical transceiver. The optical transceiver includes a modulator and/or a demodulator. The modulator modulates an optical beam emitted from the first transceiver 210 or the second transceiver 220 based on electrical signals carrying the scanning data and/or electrical signals carrying the control signals to generate the first optical beam carrying the scanning data and/or the control signals. The demodulator demodulates the received second optical beams to generate the corresponding electrical signals carrying the scanning data and/or the control signals.

In some embodiments, signal transmission may be performed between the first transceiver 210 and the second transceiver 220 in the form of optical signals. For example, the first transceiver 210 is configured to emit the first optical beam carrying information related to the scanning data to the second transceiver 220. As another example, the first transceiver 210 may receive another first optical beam carrying information related to the control signals sent by the second transceiver 220. In some embodiments, optical beams between the first transceiver 210 and the second transceiver 220 may travel within a rotating plane of the rotor 211.

FIG. 3 is a schematic diagram of a transmission system of a medical device according to some embodiments of the present disclosure.

The rotor 211 is a rotating component in the medical device (e.g., CT devices). Taking the medical device 110 is a CT device as example, the rotor 211 drives an X-ray tube and a detector to synchronously rotate, and scanning data of a target object is obtained according to the cooperation between the X-ray tube and the detector.

The rotor 211 includes a rotor body, a rotary bearing, a transmission device, and other parts. The rotor body of the rotor 211 plays a supporting role (e.g., supporting the rotating bearing, the transmission device, etc.) and is usually made of high-strength metal materials, such as stainless steel. The rotary bearing is configured to realize the rotation of the rotor 211 and may be made of ball bearings, roller bearings, or the like. The transmission device is configured to drive the rotation of the rotor 211. The transmission device includes a motor and a transmission mechanism.

In some embodiments, the first transceiver 210 is coupled to a data acquisition module of the detector on the rotor 211 to obtain the scanning data acquired by the detector from the data acquisition module.

In some embodiments, the data acquisition module, such as a data acquisition board (e.g., a Printed Circuit Board Assembly (PCBA)), is located between the first transceiver 210 and the detector. The data acquisition module is connected to the detector for receiving the scanning data acquired by the detector and transmitting the scanning data to the first transceiver 210 so that the first transceiver 210 sends the scanning data to the second transceiver 220.

In some embodiments, the transmission system 200 includes a plurality of first transceivers 210. The data acquisition module divides the scanning data acquired by the detector into a plurality of packets so as to transmit via the plurality of first transceivers 210. The plurality of packets are transmitted to the second transceiver 220, separately. The second transceiver 220 receives the plurality of packets and combines the plurality of packets.

In some embodiments, the data acquisition module may also transmit the complete scanning data acquired by the detector to the second transceiver 220 through each of the first transceivers 210 separately, so that the second transceiver 220 can receive the complete scanning data, thereby avoiding the problem of data loss during data transmission, and improving the accuracy of data transmission, and reduce a count of the second transceivers to save costs.

In some embodiments, the first transceiver 210 converts the scanning data into a first signal and transmits the first signal to the second transceiver 220. In some embodiments, the first signal may be an optical beam, an electromagnetic wave signal, or the like. The first transceiver 210 may be a first optical transceiver, or a first electromagnetic wave transceiver. Accordingly, the second transceiver 220 may be a second optical transceiver, or a second electromagnetic wave transceiver. It should be noted that the types of the first transceiver 210 and the second transceiver 220 should be consistent.

In some embodiments, the scanning data may include scanning raw data acquired by the detector. Alternatively, the scanning data may include processed raw data by a data preprocessing process. Exemplary data preprocessing process includes data smoothing, denoising, filtering, correction, reconstruction, or the like.

In some embodiments, the first transceiver 210 sends the scanning data collected by the detector to the second transceiver 220 in the form of the first signal to realize the transmission of the scanning data. In some embodiments, the first transceiver 210 also receives the control signals sent by the second transceiver 220, thereby realizing the control of the medical device by an external device.

The second transceiver 220 is configured to transmit the control signals to and receive the scanning data from the first transceiver 210 on the rotor 211. For example, the second transceiver 220 may act as a transmitter to transmit the control signals to the first transceiver 210. As another example, the second transceiver 220 may act as a receiver to receive the scanning data transmitted by the first transceiver 210.

In some embodiments, the control signals include a first instruction and/or a second instruction. The first instruction is configured to control the scanning process. For example, the first instruction includes a control instruction for controlling the medical device 110, control parameters of the medical device 110 for scanning a target object, etc. The second instruction is configured to control the transmission system 200 of the medical device 110. For example, the second instruction includes control parameters for controlling the rotor 211 to start or stop rotating, a rotating speed instruction of the rotor 211, etc.

The stator 221 is a fixed structure of the medical device 110. For example, in the CT device, the stator 221 may be a fixed structure located at an inner side of a gantry of the CT device.

In some embodiments, the stator 221 may include components such as a gantry control circuitry, portions of a communication circuitry, drive motors, power distribution, tilt hydraulics, cooling components, or the like.

The stator 221 may provide a fixed support and a base frame for the medical device 110 to carry and secure a plurality of components and circuits that are not involved in the rotation. At the same time, the stator 221 provides necessary power, the control signals, and support conditions for the rotation of the rotor 211, thereby ensuring that the rotor 211 can rotate stably and precisely.

In some embodiments, the stator 221 may be mounted outside a circumference of the rotor 211. For example, as shown in FIG. 3, the stator 221 may be mounted in a space between an inner side of the gantry 111 and the outer side of the circumference 340 of the rotor 211. More detailed descriptions may be found in FIG.3 to FIG.8B of the present disclosure and their related descriptions.

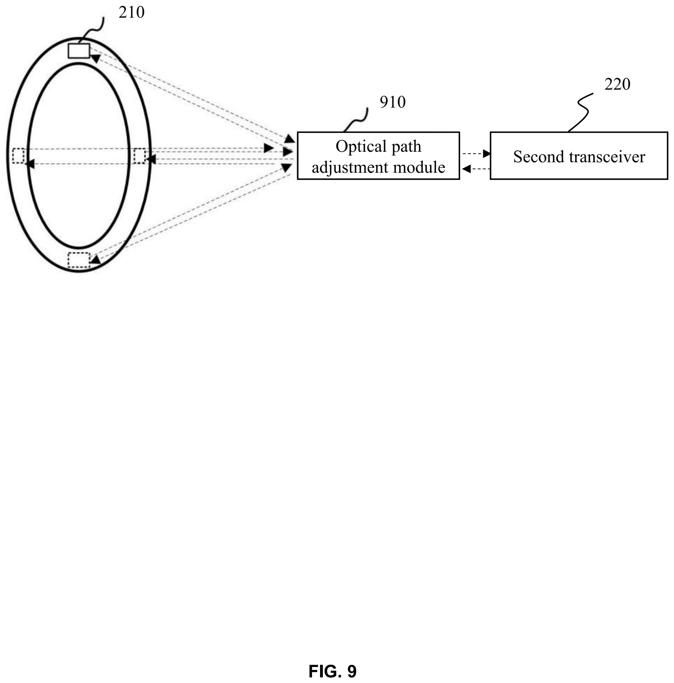

In some embodiments, the stator 221 may be located at a rotational axis of the rotor 211. More detailed descriptions may be found in FIG.9 to FIG.16 of the present disclosure and their related descriptions.

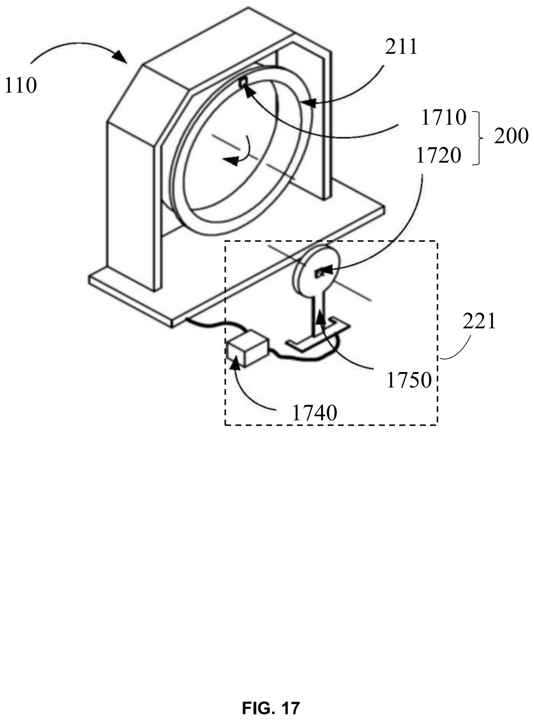

In some embodiments, the stator 221 may also be located in other locations. For example, a position set within a signal transmission range of the first transceiver 210, etc. More detailed description may be found in FIG.17 to FIG.21 of the present disclosure and their related descriptions.

The stator 221 may also be referred to as a stationary section or fixed part, and the rotor 211 may also be referred to as a rotating section or rotating part.

In some embodiments, the second transceiver 220 may be located at the rotational axis of the rotor 211 or located within a preset range of the rotational axis of the rotor 211. The second transceiver 220 may be independent of the gantry of the medical device and located outside of the gantry. For example, the second transceiver 220 may be located at a side that is back from the scanning bed and within a preset distance from a rotation center of the rotor 211. A distance between the second transceiver 220 and the rotation center is less than a distance of movement of the scanning bed into the gantry, to avoid the location of the second transceiver 220 affecting the movement of the scanning bed.

In some embodiments, the second transceiver 220 is coupled with an external device to receive the control signal sent by the external device and convert the control signal into a second signal. The second signal is transmitted to the first transceiver 210. In some embodiments, the second signal may be an optical beam, an electromagnetic wave signal, or the like. The first transceiver 210 may be coupled with a controller of the medical device to send the second signal to the controller of the medical device to indicate the controller to perform control of the medical device.

In some embodiments, the first transceiver 210 may include a first transmitter and a first receiver. The second transceiver 220 may include a second transmitter and a second receiver. The first transmitter in the first transceiver 210 may be coupled to a data acquisition module of the detector to obtain the scanning data, and convert the scanning data into the first signal. The first signal is transmitted to the second transceiver 220. The second receiver in the second transceiver 220 receives and parses the first signal. An image reconstruction is performed based on the scanning data to obtain a reconstructed image. Exemplarily, the second receiver in the second transceiver 220 may be connected to a reconstruction host to send the scanning data to the reconstruction host, and the reconstruction host performs the image reconstruction to obtain the reconstructed image.

In some embodiments, the second transmitter in the second transceiver 220 may be connected to an external device to obtain the control signal from the external device and convert the control signal into a second signal. The second signal is transmitted to the first transceiver 210. The first receiver in the first transceiver 210 receives and parses the second signal. The first receiver may be connected to the controller in the medical device to send the parsed second signal to the controller of the medical device to instruct the controller to control the medical device.

Exemplarily, the external device may include a control device located in the operation room, by which a user may control the medical device in the scanning room, thereby realizing a medical scan. Exemplarily, the reconstruction host may include a control device located in the operation room, or may include a local server, a remote server, a cloud server, or the like.

In some embodiments, the bidirectional transmission between the first transceiver and the second transceiver 220 is wireless.

In some embodiments, the bidirectional transmission between the first transceiver and the second transceiver is a transmission via wave-based signals. The wave-based signals include at least one of optical waves, radio frequency waves, millimeter waves, terahertz waves, electromagnetic waves, or sound waves, etc. In some embodiments, the optical waves may include visible optical or infrared optical, etc. The electromagnetic waves may include radio frequency, terahertz, or millimeter waves, etc. The sound waves may include ultrasonic waves, etc.

For example, the bidirectional transmission between the first transceiver 210 and the second transceiver 220 is a transmission via optical waves or electromagnetic waves. More relevant descriptions of the bidirectional transmission may be found in FIG.3 to FIG.22 of the present disclosure and their related descriptions.

In some embodiments, unidirectional transmission between the first transceiver 210 and the second transceiver 220 is also achieved via the optical waves or the electromagnetic waves. For example, the first transceiver 210 only transmits the scanning data to the second transceiver 220 via the optical waves or the electromagnetic waves. As another example, the second transceiver 220 only transmits the control signals to the first transceiver 210 via the optical waves or the electromagnetic waves.

In some embodiments, the first transceiver and the second transceiver 220 are capable of realizing a bidirectional wireless transmission without cables, which can effectively reduce the size of the transmission system, simplify the physical structure of the transmission system, and make the integration of the transmission system convenient.

In some embodiments, a bidirectional transmission between the first transceiver 210 and the second transceiver 220 is a transmission via optical waves. For example, one of the first transceiver 210 and the second transceiver 220 is a transmitter to transmit scanning data or control signals via a first optical beam to the other. The other of the first transceiver 210 and the second transceiver 220 is a receiver to receive the first optical beam.

In some embodiments, the transmission system 200 includes a plurality of first transceivers or a plurality of second transceivers. The plurality of first transceivers or the plurality of second transceivers are arranged along a circumference of the rotor and/or the stator.

As shown in FIG. 3, the transmission system 200 includes the first transceiver 210, the second transceiver 220, and a beam expander 230.

In some embodiments, as shown in FIG. 3, the first transceiver 210 is located at the circumference 240 of the rotor 211, and the stator 221 is located outside the circumference 240 of the rotor 211.

The circumference 240 of the rotor 211 refers to an outermost annulus of the rotor 211.

In some embodiments, the first transceiver 210 or the second transceiver 220 is configured to emit the first optical beam along a rotating plane of the rotor 211.

The rotating plane refers to a geometric plane formed by the rotor 211 as the rotor 211 rotates at a high speed. In some embodiments, the rotating plane is a plane where the circumference 240 of the rotor 211 is located.

The first optical beam is a beam emitted by one of the first transceiver 210 or the second transceiver 220 that acts as the transmitter. In some embodiments, the data carried by the first optical beam may include scanning data and/or scanning instructions.

In some embodiments, when the first transceiver 210 located at the rotor 211 emits the first optical beam, the first optical beam records or includes the scanning data (e.g., CT scanning data) acquired by the medical device 110. In some embodiments, when the second transceiver 220 located at the stator 221 emits the first optical beam, the first optical beam records or includes the control signal for controlling the medical device 110. The processor 140 may convert the scanning data or the control signal into the first optical beam. The first optical beam is then output from the first transceiver 210 or the second transceiver 220.

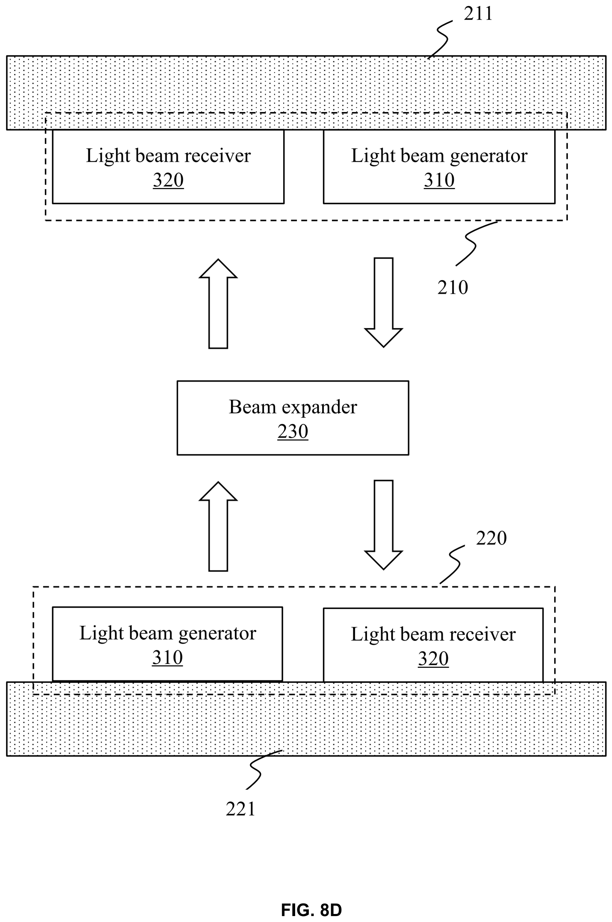

In some embodiments, when the first transceiver 210 located at the rotor 211 and the second transceiver 220 located at the stator 221 emit a first optical beam simultaneously or at intervals, the first optical beams may respectively record scanning data and scanning instructions. For example, the processor 140 may convert the scanning data into a first optical beam and emit, via the first transceiver 210 located at the rotor 211, to the second transceiver 220. As another example, the processor 140 may convert the control signals into another first optical beam and emit, via the second transceiver 220 located at the stator 221, to the first transceiver 210. Further description of related content may be found with reference to FIG. 8D.

In some embodiments, the first transceiver 210 or the second transceiver 220 is configured to receive at least a portion of the plurality of second optical beams. The second optical beams may be a plurality of beams of different wavelengths that are split from the first optical beam by the beam expander 230.

In some embodiments, a distance between the first transceiver 210 and the second transceiver 220 may be preset.

In some embodiments, the distance between the first transceiver 210 and the second transceiver 220 is proportional to a power of a beam emitted by the first transceiver 210 (or the second transceiver 220). For example, the greater the power, the greater the distance between the first transceiver 210 and the second transceiver 220.

In some embodiments, the distance between the first transceiver 210 and the second transceiver 220 is adjustable. For example, the distance between the first transceiver 210 and the second transceiver 220 may be adjustable based on the power of the beam emitted by the first transceiver 210 (or the second transceiver 220). The greater the power, the greater the adjusted distance.

In some embodiments, the distance between the first transceiver 210 and the second transceiver 220 may be as small as possible to reduce a size of the medical device.

In some embodiments, in order to balance the size of the medical device and the power of the emitted signal, the distance between the first transceiver 210 and the second transceiver 220 may be less than 20 cm. For example, the distance between the first transceiver 210 and the second transceiver 220 may be in a range of 10 cm to 20 cm. As another example, the distance between the first transceiver 210 and the second transceiver 220 may be within a range of 12 cm to 15 cm.

The beam expander 230 refers to a device for amplifying an optical spot area of an input optical beam. For example, an optical spot area of an output optical beam (or a total area of a plurality of output optical beams) of the beam expander 230 is greater than an optical spot area of an input optical beam (or a total area of a plurality of input optical beams) of the beam expander 230. In some embodiments, the beam expander 230 is configured to divide the first optical beam emitted by the first transceiver 210 (or the second transceiver 220) into a plurality of second optical beams.

In some embodiments, the plurality of second optical beams may have different wavelengths (e.g., wavelength 1, wavelength 2, wavelength 3, as shown in FIG. 3). The second optical beams with different wavelengths avoid mutual interference during transmission. In some embodiments, the plurality of second optical beams may have identical wavelengths. The second optical beams with identical wavelengths facilitate data processing (e.g., merging). In some embodiments, the plurality of second optical beams have different wavelengths, thereby balancing interference reduction and data processing facilitation.

In some embodiments, the beam expander 230 may include a diffusion lens. In some embodiments, the beam expander 230 may include a wavelength division demultiplexer and a filter.

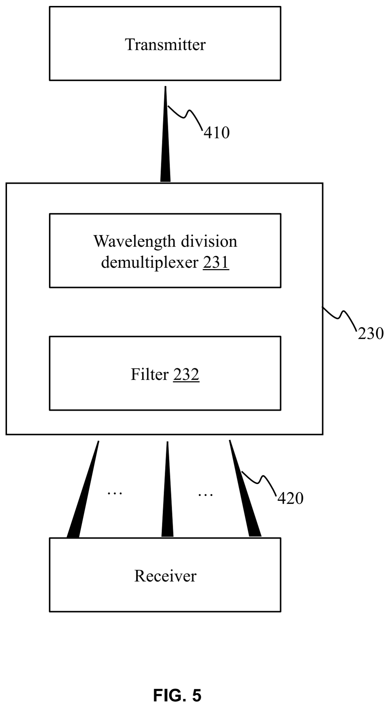

A second optical beam of the plurality of second optical beams is a beam obtained after the first optical beam has been expanded. In some embodiments, the second optical beams are converted into an electrical signal to obtain the scanning data or the control signal. Further description of the second optical beams may be found in FIG. 5 and relevant descriptions.

In some embodiments, each of the plurality of second optical beams has a same optical spot area and records at least part of same data as the first optical beam. For example, the first optical beam records data 1, data 2, and data 3. The plurality of second optical beams includes a second optical beam 1 and a second optical beam 2. The second optical beam 1 records data 1 and data 2, and the second optical beam 2 records data 3. As another example, the first optical beam records data 1, data 2, and data 3. Each of the plurality of second optical beams records data 1, data 2, and data 3. The second optical beam has the same spot area as the first optical beam refers that the first optical beam and the second optical beam have an identical beam diameter. It should be noted that during optical transmission, beam diameters cannot be perfectly identical due to diffraction effects. In the embodiments, the same spot area refers that a beam diameter error between the second optical beam and the first optical beam is less than a preset threshold. For example, the beam diameter error is less than 10%. As another example, the beam diameter error is less than 8%. As yet another example, the beam diameter error is less than 5%. In some embodiments, spot areas of part of the plurality of second optical beams may also differ from that of the first optical beam.

In some embodiments, each second optical beam carries data identical to the first optical beam. For example, scanning data and/or scanning instructions carried by each second optical beam are identical to those of the first optical beam. Exemplarily, when the first optical beam carries the scanning data, scanning data carried by each second optical beam is identical to that of the first optical beam. When the first optical beam carries the scanning instructions, scanning instructions carried by each second optical beam are identical to those of the first optical beam. During bidirectional data transmission between the stator 221 and the rotor 211, the rotor 211 transmits the first optical beam carrying the scanning data. The stator 221 transmits the first optical beam carrying the scanning instructions. Correspondingly, each second optical beam received by the stator 221 carries the scanning data identical to the first optical beam. Each second optical beam received by the rotor 211 carries the scanning instructions identical to the first optical beam.

In some embodiments, by amplifying the first optical beam to obtain the plurality of second optical beams, a count of the first transceivers 210 and/or the second transceivers 220 may be reduced to reduce the size of the medical device while ensuring the authenticity and integrity of the scanning data or control signal. Furthermore, by configuring the expanded second optical beams to have a same spot area and carry identical data as the first optical beam, issues of spot deformation or data distortion caused by beam splitting are prevented..

In some embodiments, each second optical beam of the plurality of second optical beams are independent. That is, optical spots of the plurality of second optical beams are mutually independent. The term "independent" refers to no mutual interference among the plurality of second optical beams. For instance, among the plurality of second optical beams emitted by the beam expander 230, if one or some second optical beams break down, such as being turned-off, deformed, etc., the remaining second optical beams remain unaffected and continue transmission unimpeded. In some embodiments, independent optical spots of the second optical beams prevent impacts on other independent second optical beams when partial optical spots experience extinction, deformation, or data distortion. This enhances signal-to-noise ratio and improves subsequent imaging quality.

In some embodiments, the plurality of second optical beams are emitted in a radial direction along the circumference of the rotor 211.

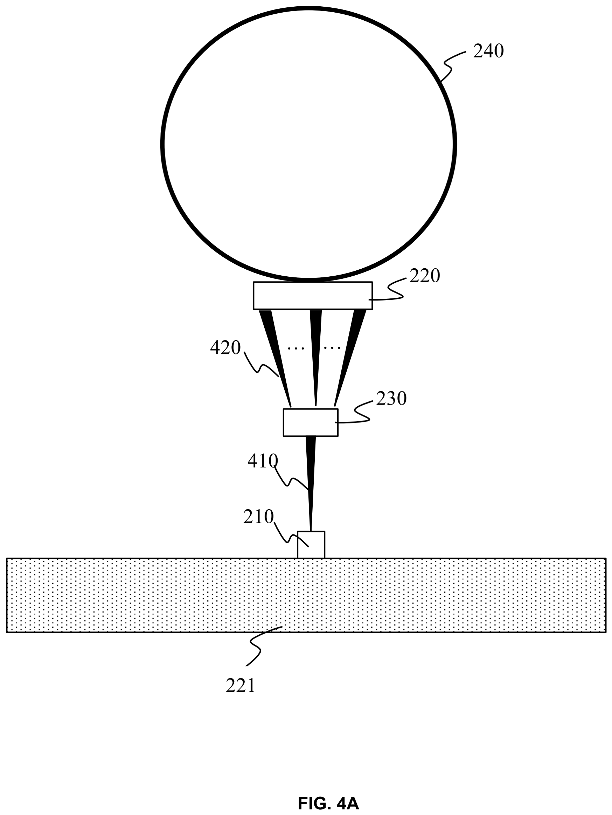

FIG. 4A is an exemplary schematic diagram of a transmission system of a medical device according to some other embodiments of the present disclosure. In some embodiments, as shown in FIG. 4A, when the first transceiver 210 acts as a transmitter, the first transceiver 210 located on the stator 221 emits the first optical beam 410. The first optical beam 410 passes through the beam expander 230 to be divided into a plurality of second optical beams 420 with different wavelengths. The plurality of second optical beams 420 of different wavelengths are received by the second transceiver 220 located on the circumference 240 of the rotor. The second transceiver 220 receives at least a portion of the plurality of second optical beams 420 with different wavelengths.

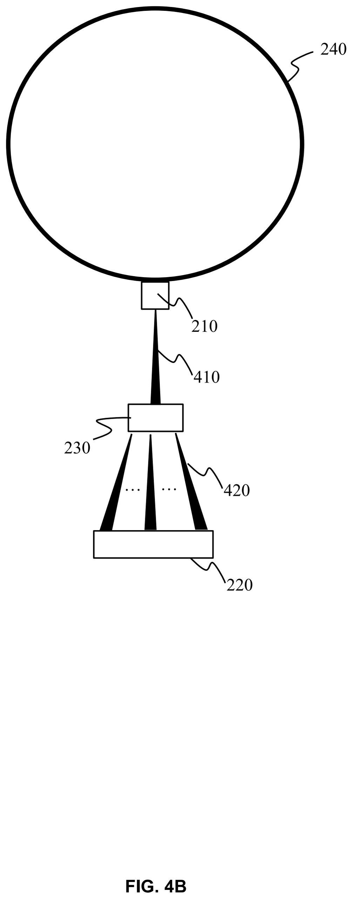

FIG. 4B is a schematic diagram of an exemplary arrangement of second optical beams according to some embodiments of the present disclosure.

As shown in FIG. 4B, when the first transceiver 210 acts as a transmitter, the first transceiver 210 located at the circumference 240 of the rotor 210 transmits the first optical beam 410. The first optical beam 410 passes through the beam expander 230 to be divided into a plurality of second optical beams 420 of different wavelengths. The plurality of second optical beams 420 of different wavelengths are emitted in the radial direction along the circumference 240. The second transceiver 220 receives at least a portion of the plurality of second optical beams 420 of different wavelengths.

In some embodiments, when the second transceiver 220 acts as a transmitter (not shown in the figure), the beam propagation and expansion process is similar to that the first transceiver 210 acts as the transmitter. Exemplarily, the second transceiver 220 is located at the stator 220 to emit the first optical beam. The first optical beam is divided into a plurality of second optical beams of different wavelengths by the beam expander 230. The plurality of second optical beams of different wavelengths are emitted in the radial direction along the circumference of the rotor 210. The first transceiver 210 receives at least part of the plurality of second optical beams of different wavelengths. In some embodiments, when the second transceiver 220 acts as a transmitter (not shown in the figure), the beam propagation and expansion process is not same as that the first transceiver 210 acts as the transmitter. For example, the second transceiver 220 located at the stator 220 directly emits the first optical beam (without dividing into a plurality of second optical beams) to the first transceiver 210.

In some embodiments, the plurality of the second optical beams of different wavelengths are emitted in the radial direction along the circumference of the rotor, and an optical spot area of the optical beams received by the receiver in the first transceiver 210 or the second transceiver 220 may be as large as possible to obtain sufficiently large amounts of the scanning data or the control signal to avoid data loss and distortion.

FIG. 5 is a schematic diagram of a transmission system of a medical device according to some embodiments of the present disclosure.

As shown in FIG. 5, the beam expander 230 includes a wavelength division demultiplexer 231 and a filter 232.

The wavelength division demultiplexer 231 is configured to separate the first optical beam into a plurality of sub-beams of different wavelengths. Each sub-beam has a same optical spot area and recorded scanning data as the first optical beam.

The filter 232 is configured to filter the plurality of second optical beams. In some embodiments, the filter 232 includes a plurality of filtering elements. Each of the plurality of filtering elements corresponds to a required wavelength of the plurality of second optical beams. For example, the first optical beam is input into the plurality of filtering elements of the filter 232, and the filter 232 outputs the plurality of second optical beams, each of which has a required wavelength.

In some embodiments, the filter 232 is mounted on the circumference of the rotor 211. With the rotation of the rotor 211, the plurality of second optical beams are emitted in the radial direction along the circumference of the rotor 211.

In some embodiments, the filter 232 is a ring-shaped filter. The ring-shaped filter is an optical device based on a ring resonance structure to achieve selective filtering of specific wavelengths through resonance effect of optical in a ring cavity. The ring-shaped filter is configured to utilize the resonance effect of the ring cavity combined with the waveguide coupling mechanism, to achieve narrow bandwidth, high transmittance, and tunable filtering.

In some embodiments, the ring-shaped filter is configured to control spatial distribution of the second optical beams, thereby enabling the second optical beams to be emitted in the radial direction along the circumference of the rotor 211.

In some embodiments, the ring-shaped filter is further configured to filter optical beams output by the wavelength division demultiplexer 231 to obtain the plurality of second optical beams 420.

In some embodiments, as shown in FIG. 5, the transmitter transmits the first optical beam 410 to the beam expander 230. The wavelength division demultiplexer 231 in the beam expander 230 divided the first optical beam 410 into a plurality of wavelengths of second optical beams, and controls spatial distribution of the plurality of second optical beams to enable arrangement of the second optical beams 420 side by side along the tangential direction of the circumference of the rotor 211. Thus, the second transceiver 220 receives at least part of the plurality of second optical beams of different wavelengths.

In some embodiments, the wavelength division demultiplexer divides the first optical beam using an interval of 1 nm (e.g., the wavelength interval between adjacent second optical beams obtained by dividing the first optical beam is 1 nm), to be applied to ultra-high-speed data transmission (e.g., >100 Gbps). In some embodiments, the wavelength division demultiplexer divides the first optical beam using an interval of 5 nm, to balance bandwidth and cost for medium-speed data transmission (e.g., 50 Gbps). In some embodiments, the wavelength division demultiplexer divides the first optical beam using an interval of 20 nm, to be applied to low-cost data transmission scenarios (e.g., <25 Gbps).

The transmitter, the receiver are one of the first transceiver 210 or the second transceiver 220, respectively. For example, when the first transceiver 210 is the transmitter, then the second transceiver 220 is the receiver. Alternatively, when the first transceiver 210 is the receiver, then the second transceiver 220 is the transmitter.

In some embodiments, one or more second transceivers 220 may be provided to receive the plurality of second optical beams. For example, the beam expander 230 divides the first optical beam with a wavelength A into a certain count (e.g., B) of second optical beams. Each of the B second optical beams has a wavelength A/B (A÷B). The second transceiver 220 receives the B second optical beams and combines the B second optical beams to obtain a total wavelength A. As another example, the beam expander 230 divides the first optical beam with a wavelength A into B second optical beams. Each of the B second optical beams has the same wavelength A. B second transceivers 220 are provided, and each of the B second transceivers 220 receives one of the B second optical beams with the wavelength A.

In some embodiments, after receiving the plurality of second optical beams, the second transceiver 220 can combine the plurality of second optical beams into one beam and then send the combined beam to a subsequent electrical processing module (e.g., a module for photoelectric converter) (not shown in the figure). After receiving the plurality of second optical beams, the second transceiver 220 directly sends the received the plurality of second optical beams to the electrical processing module. The electrical processing module combines and processes (e.g., performing data optimization, performing image reconstruction) the plurality of second optical beams.

In some embodiments, the second receiver 220 may also include a filter (not shown in the figure) to filter the plurality of second optical beams. For example, the filter is configured to remove or filter unnecessary information (e.g., overlapping parts in data carried by different second optical beams or some other unwanted clutter data)) in the plurality of second optical beams. The overlapping part refers to same data carried by the plurality of second optical beams. For example, the data carried by the first light beam includes data packets 1-20. The first light beam is divided into three second light beams by the beam expander, such as a second light beam 1, a second light beam 2, and a second light beam 3. The second light beam 1 carries data packets 1-8, the second light beam 2 carries data packets 8-14, and the second light beam 3 carries data packets 13-20. Data packet 8 is the overlapping part of the data of the second light beam 1 and the second light beam 2. After being filtered by the filter, only one data packet 8 is retained. Data packet 13 and data packet 14 are the overlapping parts of the data of the second light beam 2 and the second light beam 3. After being filtered by the filter, only one data packet 13 and one data packet 14 are retained.

In some embodiments, the beam expander 230 expands the first optical beam based on a wavelength division demultiplexer and a ring-shaped filter, which not only realizes the simplification of the optical path, but also carries a larger bandwidth. Taking photon counting medical devices as an example, which have a relatively large amount of data, the bandwidth of signals propagated by traditional beam expansion manners (e.g., diffusion lenses) is small, often only tens of Gbps, whereas expanding the beam through the wavelength division demultiplexer and the ring-shaped filter can achieve bandwidths of up to 100 Gbps for each optical spot (or for each wavelength corresponding to an optical spot). Furthermore, after the wavelength division demultiplexer divides the first optical beam into the plurality of wavelength second optical beams, the ring-shaped filter controls propagation directions of the second optical beams via a resonance effect of its ring cavity. All second optical beams are emitted in the radial direction along the circumference of the rotor within a rotating plane while ensuring each second optical beam maintains an identical spot diameter as the first optical beam. Thereby, issues of spot deformation or data distortion caused by beam separation are resolved.

In some embodiments, by locating the first transceiver 210 at the rotor on the circumference of the rotor, and the second transceiver 220 and the stator outside the circumference of the rotor, a compact structure inside the medical device is achieved, and the size of the medical device is reduced. In addition, by utilizing WDM technology by the beam expander 230, an optical spot containing data emitted by a transmitter is divided into a plurality of optical spots with different wavelengths, each of the plurality of optical spots contains the same data as the original emitted optical spot and has the same optical spot area, only with different wavelengths. It is equivalent to diffusing the original one optical spot into a plurality of optical spots, which are then emitted to the receivers, to ensure that a small count of receivers (instead of a large count of receiver arrays) can receive complete data, further reducing the size of the medical device while ensuring the integrity and authenticity of the data/instructions. Further, different wavelengths are transmitted simultaneously to reduce interference during data transmission.

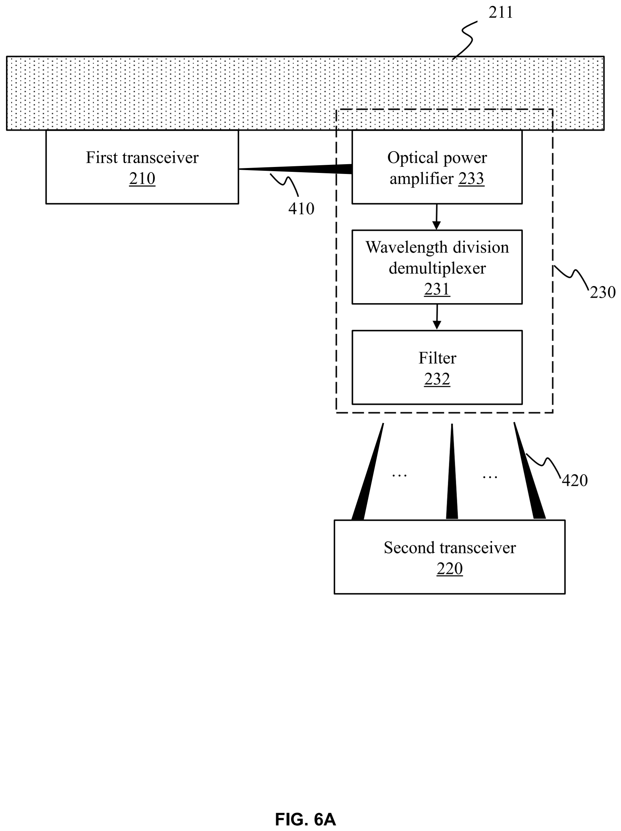

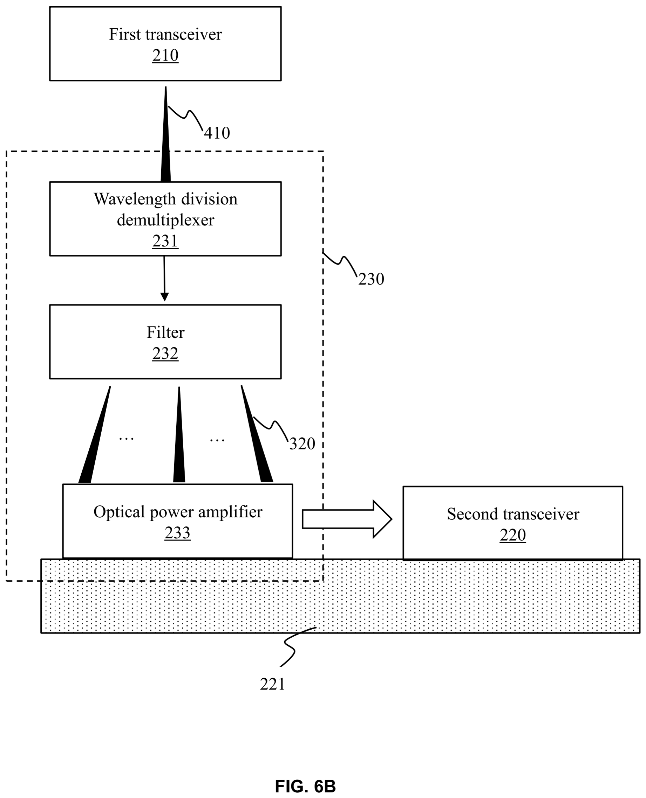

FIG. 6A is a schematic diagram of an exemplary arrangement of an optical power amplifier according to some embodiments of the present disclosure. FIG. 6B is a schematic diagram of an exemplary arrangement of an optical power amplifier according to some other embodiments of the present disclosure.

In some embodiments, as shown in FIG. 6A and FIG. 6B, the beam expander 230 further includes an optical power amplifier 233.

The optical power amplifier 233 may amplify the power of the optical beam, reduce the loss of power of the optical beam due to the wavelength division demultiplexer and the ring-shaped filter, and improve the quality of the subsequent CT scan images.

In some embodiments, the optical power amplifier 233 may be located at an input end where the first optical beam enters the wavelength division demultiplexer and/or an output end where the second optical beams exit the wavelength division demultiplexer. For example, during data transmission from the rotor 211 to the stator 221, the optical power amplifier 233 may be located at the rotor 211 to amplify the optical power of the first optical beam. Alternatively, the optical power amplifier 233 may be located at the stator 221 to amplify the optical power of the second optical beams. For another example, during data transmission from the stator 221 to the rotor 211, the optical power amplifier 233 may be located at the stator 221 to amplify the optical power of the first optical beam. Alternatively, the optical power amplifier 233 may be located at the rotor 211 to amplify the optical power of the second optical beams. For yet another example, during bidirectional data transmission, the optical power amplifier 233 may be located at the stator 221 and/or the rotor 211. In some embodiments, the optical power amplifier 233 is not located at the rotor 211 or stator 221. For instance, the optical power amplifier 233 may be located along an optical path between the rotor 211 and stator 221 (e.g., inside or outside the transmission system) and connected to the wavelength division demultiplexer 231 via cables or similar means (e.g., connected to an input end and/or output end of the wavelength division demultiplexer 231).

In some embodiments, the optical power amplifier 233 may be connected to the input end of the wavelength division demultiplexer 231 to amplify the optical power of the first optical beam 410 emitted by the first transceiver 210. The input end of the wavelength division demultiplexer 231 refers to an end where the first optical beam enters the wavelength division demultiplexer 231, as shown in FIG. 6A.

In some embodiments, as shown in FIG. 6B, the optical power amplifier 233 may be connected to the filter 232 (or an output end of the wavelength division demultiplexer 231) to amplify the optical power of each second optical beam 420 or the power of part of the second optical beams 420 (e.g., selectively amplifying the power of specific-wavelength second optical beams). The output end of the wavelength division demultiplexer 231 refers to an end where the second optical beams exit the wavelength division demultiplexer.

In some embodiments, a plurality of optical power amplifiers may be provided. The plurality of optical power amplifiers may be located at the end where the first optical beam enters the wavelength division demultiplexer and the end where the second optical beams exit the wavelength division demultiplexer. For example, an optical power amplifier may be simultaneously connected to both the input end and the output end of the wavelength division demultiplexer (or the ring-shaped filter) to amplify optical power of the first optical beam and optical power of each or part of the second optical beams.

It should be noted that the beam expander 230 is composed of a plurality of different components, and FIG. 2 to FIG. 6B are only examples for illustrating the location of the beam expander. The different components of the beam expander 230 may be located at different positions. For example, the wavelength division demultiplexer and the ring-shaped filter may be located at the rotor 211, and the optical power amplifier is located at the stator 221. As another example, the wavelength division demultiplexer, the ring-shaped filter, and the optical power amplifier may all be located at the rotor 211. In addition, FIG. 2 to FIG. 6B only show a case in which the first transceiver 210 serves as a transmitter and the second transceiver 220 serves as a receiver, and when the first transceiver 210 serves as a receiver and the second transceiver 220 serves as a transmitter, the optical power amplifier 223 still works similarly.

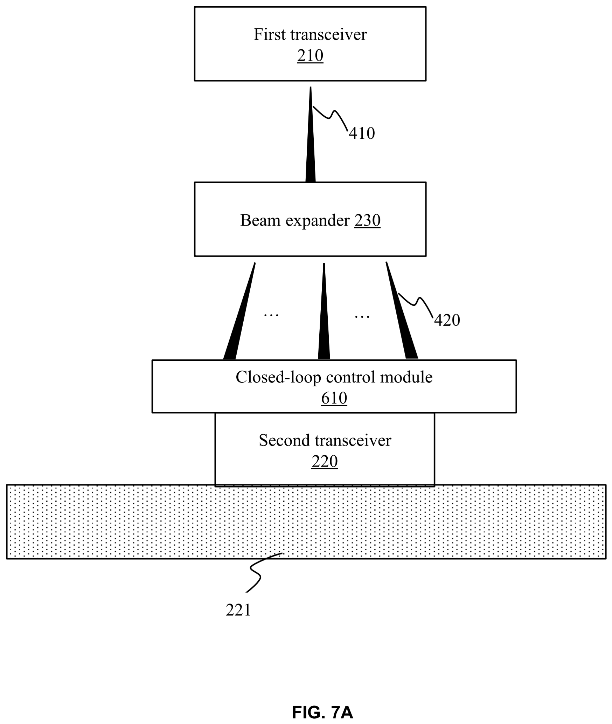

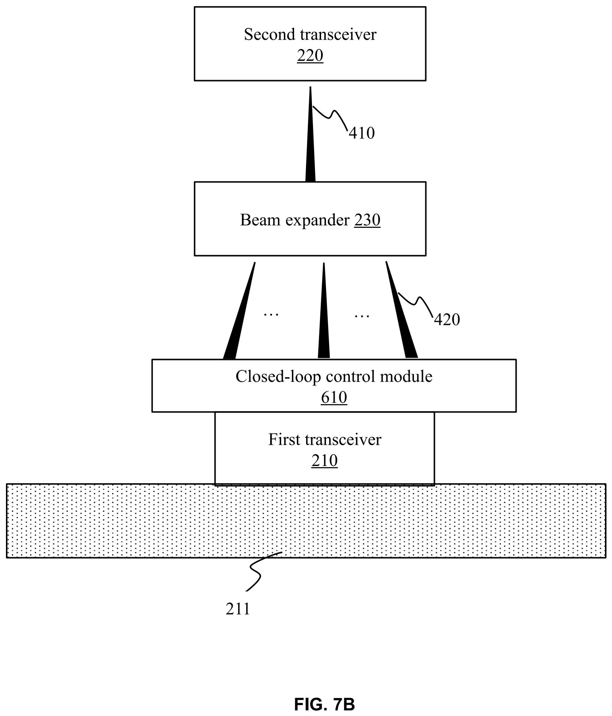

FIG. 7A is a schematic diagram of an exemplary arrangement of a closed-loop control module according to some embodiments of the present disclosure. FIG. 7B is a schematic diagram of an exemplary arrangement of an angle determination module according to some other embodiments of the present disclosure.

As shown in FIG. 7A and FIG. 7B, the transmission system 200 may further include a closed-loop control module 610.

In some embodiments, the transmission system 200 of the medical device further includes a closed-loop control module 610 located at the stator 221 and/or the rotor 211. In some embodiments, when the first transceiver 210 located at the rotor 211 transmits data to the second transceiver 220 located at the stator 221, the closed-loop control module 610 may be located at the stator 221 (or at an output end of the beam expander 230). In some embodiments, when the second transceiver 220 located at the stator 221 transmits data to the first transceiver 210 located at the rotor 211, the closed-loop control module may be located at the rotor 211 (or at an output end of the beam expander 230) (not shown). In some embodiments, when the first transceiver 210 located at the rotor 211 and the second transceiver 220 located at the stator 221 transmit data to each other (e.g., the bidirectional transmission between the first transceiver 210 and the second transceiver 220), the closed-loop control module may be located at both the stator 221 and the rotor 211 (not shown). That is, a closed-loop control module is necessarily located at an end where the second optical beams exit the beam expander to monitor received optical power in real-time.

The closed-loop control module 610 is configured to monitor an optical power of a received optical beam in real-time and dynamically adjust an arrangement parameter. The arrangement parameter includes an arrangement direction, a count of the plurality of second optical beams, etc. In some embodiments, the closed-loop control module 610 may include an optical sensor, an optical power sensor, or the like.

In some embodiments, the closed-loop control module 610 may monitor the optical power of the received optical beam and adjust the optical power. For example, when the optical power received by the closed-loop control module 610 is too strong (e.g., exceeding a first preset optical power threshold), the transmitter (e.g., the first transceiver 210 or the second transceiver 220 ) may be controlled to reduce the power of emitted optical beam, or the optical power amplifier 233 may be controlled to reduce the amplified optical power. As another example, when the optical power received by the closed-loop control module 610 is too low (e.g., being less than a preset second optical power threshold), the transmitter (e.g., the first transceiver 210 or the second transceiver 220 ) may be controlled to enhance the power of the emitted beam, or the optical power amplifier 233 may be controlled to increase the amplified optical power.

In some embodiments, the closed-loop control module 610 may dynamically adjust the arrangement parameter. For example, when the closed-loop control module 610 receives too few optical spots (e.g., being less than a preset optical spot count threshold) of the second optical beams, indicating that the receiver (e.g., the first transceiver 210 or the second transceiver 220) also receives fewer beams, which may result in distortion or incompleteness of the scanning data (e.g., scanning data distortion and/or scanning instruction loss). For another example, when the closed-loop control module 610 detects abnormal directions of the received second optical beams (e.g., failure of the plurality of second optical beams to be emitted in the radial direction along the circumference of the rotor, such as beam crossing, overlapping, or spot deformation), this may also cause data distortion or incompleteness. The closed-loop control module 610 controls the wavelength division demultiplexer 231 and the filter 232 to adjust the arrangement direction and/or count of the second optical beams, thereby preventing data distortion and achieving enhanced imaging quality along with improved control effectiveness of the rotary optical transmission system.

In some embodiments, the closed-loop control module 610 may be located at the rotor 211 and/or the stator 221 of the medical device.

In some embodiments, the first transceiver 210 acts as a transmitter, and the second transceiver 220 acts as a receiver, as shown in FIG. 7A. The closed-loop control module 610 located at the stator 221 monitors the optical power of the optical beam received by the second transceiver 220 and adjusts the optical power so that the optical power of the optical beam received by the second transceiver 220 is in a suitable range (e.g., being not greater than the first preset optical power threshold and not less than the second preset optical power threshold). The closed-loop control module 610 dynamically adjusts the arrangement direction and/or the count of the plurality of second optical beams 420 output by the beam expander 230 to avoid distortion or incompleteness of the scanning data received by the second transceiver 220.

In some embodiments, as shown in FIG. 7B, the second transceiver 220 acts as a transmitter, and the first transceiver 210 acts as a receiver. At this time, the closed-loop control module 610 located at the rotor 211 monitors the optical power of the optical beam received by the first transceiver 210 and adjusts the optical power so that the optical power of the optical beam received by the first transceiver 210 is in a suitable range (e.g., being not greater than a first preset optical power threshold and not less than a second preset optical power threshold). The closed-loop control module 610 dynamically adjusts the direction and/or the count of the plurality of second optical beams 420 outputted by the beam expander 230 to avoid distortion or incompleteness of the control signal.

In some embodiments, by monitoring the received optical power in real-time by the closed-loop control module and dynamically adjusting the arrangement direction and/or the count of the plurality of second optical beams, the intensity and the count of the optical beams may be ensured, avoiding the distortion or loss of the received signals/data from the impact and improve the data transmission effect.

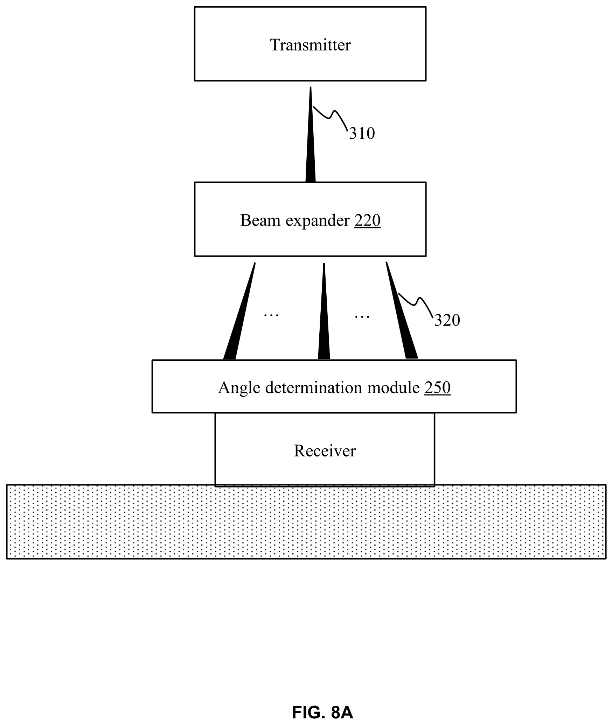

FIG. 8A is a schematic diagram of an exemplary arrangement of an angle determination module according to some embodiments of the present disclosure. It should be understood that the same modules in FIGs. 5, 6A-6B, 7A-7B, and 8A can refer to the descriptions in FIG. 5. Different modules, such as the closed-loop control module 610 in FIGs. 7A-7B and the angle determination module 250 in FIG. 8A, may be included in the transmission system 200 individually or in combination.

In some embodiments, the transmission system 200 of the medical device may further include an angle determination module (also referred to as an optical spot angle determination module) located at the stator and/or the rotor. In some embodiments, when the first transceiver 210 located at the rotor 211 transmits data to the second transceiver 220 located at the stator 221, the angle determination module 250 may be located at the stator 221 (or an output end of the beam expander 230). In some embodiments, when the second transceiver 220 located at the stator 221 transmits data to the first transceiver 210 located at the rotor 211, the angle determination module may be located at the rotor 211 (or an output end of the beam expander) (not shown). In some embodiments, when the first transceiver 210 located at the rotor 211 and the second transceiver 220 located at the stator 221 transmit data to each other (e.g., the bidirectional transmission between the first transceiver 210 and the second transceiver 220), the angle determination module may be located at both the stator 221 and the rotor 211 (not shown). That is, the angle determination module is necessarily located at an end where the second optical beams exit the beam expander to determine an angle covered by an expanded optical spot.

As shown in FIG. 8A, the transmission system 200 may further include an angle determination module 250. The angle determination module 250 may be located at the stator 221.

The angle determination module 250 is configured to determine an angle that may be covered by expanded optical spots. For example, the angle determination module 250 may determine a range of an angle that is covered by optical spots corresponding to a plurality of the second optical beams of different wavelengths.

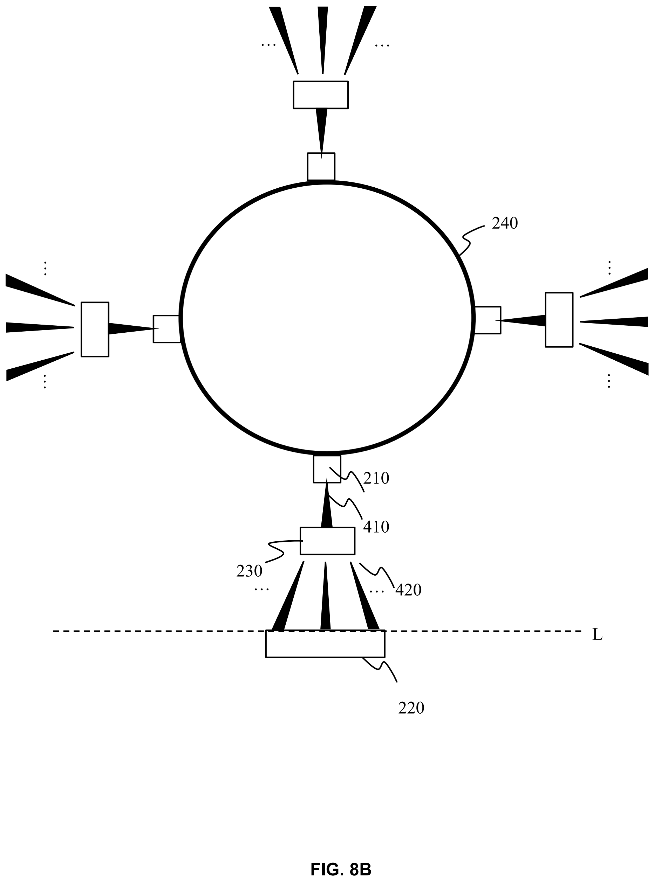

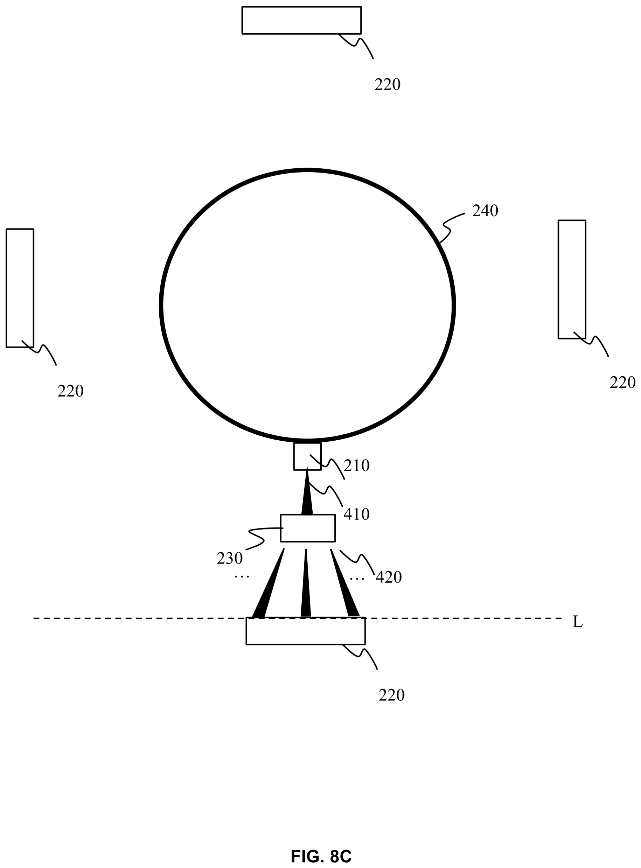

In some embodiments, the angle determination module 250 may also determine, based on the angle that is covered by the expanded optical spot, a count of the first transceivers 210 located at the circumference of the rotor 211, an interval angle between two adjacent first transceivers 210 of the first transceivers 210, a count of the second transceivers 220 located at the stator 221, and an interval angle between two adjacent second transceivers 220 of the second transceivers 220. The angle that is covered by the expanded optical spot refers to a diffusion range of the expanded optical spot on the rotating plane. The interval angle is an angle formed by a first line connecting one of an adjacent two second transceivers and a rotation center of the rotor 211 and a second line connecting the other of the adjacent two second transceivers and the rotation center of the rotor 211. A vertex of the interval angle is the rotation center. For example, after being expanded, the angle that is covered by an optical spot is 120°. One first transceiver 210 on the rotor 211 and three (i.e., 360°/120° = 3) second transceivers 220 on the stator 221 may be arranged. The interval angle between two adjacent second transceivers 220 is 120°. As another example, after being expanded, the angle that is covered by the optical spot is 120°. Three (i.e., 360°/120° = 3) first transceivers 210 on the rotor 211 and one second transceiver 220 on the stator 221 are arranged. The interval angle between two adjacent first transceivers 210 is 120°. As still another example, after being expanded, the angle that is covered by the optical spot is 90°. One first transceiver 210 on the rotor 211 and four (i.e., 360°/90° = 4) second transceivers 220 on the stator 221 are arranged. The interval angle between two adjacent second transceivers 220 is 90°, as shown in FIG. 8C. As still another example, after being expanded, the angle that is covered by the optical spot is 90°. Four (i.e., 360°/90° = 4) first transceivers 210 on the rotor 211 and one second transceiver 220 on the stator 221 are arranged. The interval angle between two adjacent first transceivers 210 is 90°, as shown in FIG. 8B.

In some embodiments, the arrangement interval angle between the first transceiver 210 and/or second transceiver 220 is less than or equal to an angle covered by the expanded optical spot (i.e., the angle covered by the plurality of second optical beams), ensuring that during rotation of the rotor 211, the second transceiver 220 continuously receives a sufficient count of optical beams to prevent data loss and distortion.

In some embodiments, the angle determination module 250 may be located adjacent to one of the first transceiver 210 and the second transceiver 220 that serves as a receiver.

In some embodiments, the transmission system 200 includes a plurality of first transceivers 210 located at the rotor 211 and a second transceiver 220 located at the stator 221. The plurality of first transceivers 210 are arranged at interval along the circumference direction (e.g., the circumference 240) of the rotor 211 according to the interval angle. At this point, the second transceiver 220 may act as the receiver, and the first transceiver 210 may act as the transmitter.

FIG. 8B is a schematic diagram of an exemplary arrangement of an angle determination module according to some other embodiments of the present disclosure.

As shown in FIG. 8B, the angle determination module 250 determines that the angle that is covered by the expanded optical spot is 90°, the angle determination module 250 may determine that four first transceivers located at the circumference of the rotor based on the angle. The four first transceivers are arranged at interval based on an interval angle of 90° (in terms of a circumferential angle of the circumference of the rotor) in a direction of the circumference of the rotor. The count of the second transceiver 220 located at the stator 221 is one. The count and the interval angle of the plurality of first transceivers in the circumference direction of the rotor are required to ensure that the second transceiver 220 can continuously receive a sufficient count of optical beams during rotation of the rotor to avoid data loss and distortion.

In some embodiments, the transmission system 200 includes the first transceiver 210 located at the rotor and a plurality of second transceivers 220 located at the stator 221. The plurality of second transceivers 220 are arranged at interval according to the interval angle along a tangential direction on the outside of the circumference 240 of the rotor.

In some embodiments, the transmission system 200 includes a plurality of first transceivers 210 located at the rotor 211 and a plurality of second transceivers 220 located at the stator 221. A count of the plurality of first transceivers 210 or a count of the plurality of second transceivers 220 is not limited, as long as a receiving angle range of the plurality of second transceivers 220 covers an emission angle sum of the plurality of first transceivers 210. For example, the receiving angle range of the plurality of second transceivers 220 covers 360°. In some embodiments, the count of the plurality of first transceivers 210 equals the count of the plurality of second transceivers 220. In some embodiments, the count of the plurality of first transceivers 210 is different from the count of the plurality of second transceivers 220.

FIG. 8C is a schematic diagram of an exemplary arrangement of an angle determination module according to some other embodiments of the present disclosure.

As shown in FIG. 8C, the angle determination module 250 determines that the angle that is covered by the expanded optical spot is 90°, and thereby determines that one first transceiver 210 located at the circumference of the rotor and the count of second transceivers 220 is four. The four second transceivers 220 are arranged at interval according to the interval angle of 90° (measured in terms of a central angle of the circumference of the rotor) on an outer side of the circumference of the rotor. The count and the interval angle of the plurality of second transceivers 220 on the outer side of the circumference of the rotor are required to ensure that the second transceivers 220 can continue to receive a sufficient count of optical beams during rotation of the rotor to avoid loss and distortion of data.

It should be noted that the FIG. 8B and FIG. 8C are only for examples, other counts of the first transceivers 210 and the second transceivers 220 may be arranged based on different angles that are covered by the expanded optical spot to avoid loss and distortion of data.

FIG. 8D is a schematic diagram of a transmission system of a medical device according to some other embodiments of the present disclosure. As shown in FIG. 8D, the transmission system 200 includes a second transceiver 220 located at the stator 221, a beam expander 230, and a first transceiver 210 located at a circumference of the rotor 211. The stator 221 is mounted outside the circumference of the rotor 211. The first transceiver 210 includes an optical beam generator 310 and an optical beam receiver 320. The second transceiver 220 includes an optical beam generator 310 and an optical beam receiver 320.

The optical beam generator 310 located at the stator 221 is configured to emit a first optical beam carrying scanning instructions along a rotating plane of the rotor 211. The beam expander 230 divides the first optical beam carrying scanning instructions into a plurality of second optical beams. The optical beam receiver 320 located at the rotor 211 receives at least part of the plurality of second optical beams of different wavelengths. In some embodiments, wavelengths of the plurality of second optical beams may be different. In some embodiments, each second optical beam has a same spot area and carries identical data as the first optical beam. For example, scanning instructions carried by each second optical beam are identical to those of the first optical beam.

The optical beam generator 310 located at the rotor 211 is configured to emit a first optical beam carrying scanning data along the rotating plane of the rotor 211. The beam expander 230 divides the first optical beam carrying scanning data into a plurality of second optical beams. The optical beam receiver 320 located at the stator 221 receives at least part of the plurality of second optical beams of different wavelengths. In some embodiments, wavelengths of the plurality of second optical beams may be different. In some embodiments, each second optical beam has an identical spot area and carries identical data as the first optical beam. For example, scanning data carried by each second optical beam is identical to that of the first optical beam.

In some embodiments, the optical beam generator 310 and the optical beam receiver 320 located at the rotor 211 may be separated devices. Alternatively, the optical beam generator 310 and the optical beam receiver 320 located at the rotor 211 may be integrated into the first transceiver 210 to both transmit to and receive data from the stator 220. The optical beam generator 310 and the optical beam receiver 320 located at the stator 220 may be separated devices. Alternatively, the optical beam generator 310 and the optical beam receiver 320 located at the stator 220 may be integrated into the second transceiver 220 to both transmit to and receive data from the rotor 211. Integrating the optical beam generator 310 and the optical beam receiver 320 into a transceiver simplifies the architecture of the transmission system, facilitating size reduction of the medical device.

In some embodiments, the beam expander 230 includes a wavelength division demultiplexer, a ring-shaped filter, and an optical power amplifier. The rotary optical transmission system 1000 further includes a closed-loop control module and an angle determination module. Detailed descriptions may be found in relevant preceding sections (e.g., FIG. 9).

In some embodiments, as shown in FIG. 8D, during bidirectional data transmission between the stator 221 and the rotor 211, the optical beam generator 310 located at the rotor 211 and the stator 221 respectively emit first optical beams. The first optical beam emitted by the optical beam generator 310 at the rotor 211 (or referred to as the first transceiver 210) carries scanning data. The first optical beam emitted by the optical beam generator 310 located at the stator 221 (or referred to as the second transceiver 220) carries scanning instructions. Correspondingly, each second optical beam received by the optical beam receiver 320at the stator 221 (or referred to as the second transceiver 220) carries scanning data identical to the first optical beam. Each second optical beam received by the optical beam receiver 320 at the rotor 211 (or referred to as the first transceiver 210) carries scanning instructions identical to the first optical beam.

**In** some embodiments, according to the angle determination module, the angle that is covered by the expanded optical spot is determined, and based on the angle, the count and the interval angle of the first transceivers 210 and/or the second transceivers 220 arranged on the rotor and/or the stator are determined. Thus, continuous transmitting and receiving of data/instructions during rotation of the rotor are ensured and the impact of data distortion and loss on data transmission during operation of the medical device is avoided.

In some embodiments, according to the angle determination module, the angle that is covered by the expanded optical spot is determined, and based on the angle, the count and the interval angle of the first transceivers 210 and/or the second transceivers 220 arranged on the rotor and/or the stator are determined. Thus, continuous transmitting and receiving of data/instructions during rotation of the rotor are ensured and the impact of data distortion and loss on data transmission during operation of the medical device is avoided.

FIG. 9 is a schematic diagram of a transmission system of a medical device according to some other embodiments of the present disclosure.

In some embodiments, a bidirectional transmission between the first transceiver 210 and the second transceiver 220 is a transmission via optical waves. For example, the first transceiver 210 may send a first signal to the second transceiver 220 via optical waves, and/or receive a second signal from the second transceiver 220 via optical waves.

As shown in FIG. 9, the transmission system 200 includes the first transceiver 210, the second transceiver 220, and an optical path adjustment module 910. The first signal is emitted in a direction from the circumference of the rotor 211 towards the rotational axis of the rotor 211.

In some embodiments, the first transceiver 210 is located on a circumference of the rotor 211, and the second transceiver 220 is located on a rotational axis of the rotor.

In some embodiments, one or more first transceivers 210 may be located at any location around the circumference of the rotor 211. For example, a plurality of first transceivers 210 are located at equal interval angle around the circumference of the rotor 211, and each of first transceivers 210 may all be oriented toward the second transceiver 220. For example, in the case where the second transceiver 220 is located at a side of a scanning bed of the medical device, each of the plurality of first transceivers 210 may be located at the side of the rotor 211 near the scanning bed. In the case where the second transceiver 220 is located at a side of the medical device back from the scanning bed, each of the plurality of first transceivers 210 may be located at the side of the rotor 211 back from the scanning bed. Thus, the signal transmission path of each of the plurality of first transceivers 210 may be oriented toward the second transceiver 220 during the rotation process of the rotor 211, thereby realizing signal transmission between the plurality of first transceivers 210 and the second transceiver 220.

A count of the first transceivers on the rotor 211 and the count of the second transceivers on the stator 221 are not limited. Further, the plurality of the first transceivers may be located at equal intervals on the rotor 211, or may be located non-equally spaced on the rotor 211. In addition, using the transmission system for the medical device involved in some embodiments of the present disclosure, a unidirectional data transmission may be realized or a bidirectional data transmission may be realized, and the transmission system may be flexibly configured according to the use requirements in actual use.

In some embodiments, the transmission system 200 may further include the optical path adjustment module 910 between the first transceiver 210 and the second transceiver 220, so as to ensure that the first transceiver 210 located at the rotor 211 may always point to the second transceiver 220 on the rotational axis during the rotation of the rotor 211. For example, the first signal sent by the first transceiver 210 needs to be projected to the second transceiver 220, and the second signal sent by the second transceiver 220 needs to be projected to the first transceiver 210, the optical path adjustment module 910 between the first transceiver 210 and the second transceiver 220 may ensure that the reliability and accuracy of the bidirectional signal transmission path.

In some embodiments, the optical path adjustment module 910 may include an adjustment unit. For example, one or two adjustment units may be located in the first transceiver 210 or the second transceiver 220, respectively.

The adjustment unit is configured to adjust the direction of signals sent to or received by the first transceiver 210 or the second transceiver 220.

In some embodiments, the optical path adjustment module includes a direction-adjusting element. The direction-adjusting element includes an optical wedge, a reflector, or a diffractive grating, etc.

In some embodiments, the optical path adjustment module may include a rotatable optical wedge. For example, the adjusting component or the adjustment unit in the optical path adjustment module may be a rotatable optical wedge.

The optical wedge is a device for changing the direction of propagation of an optical beam. In some embodiments, the optical wedge may include at least one of a planar optical wedge, a concentrating optical wedge. For example, when the optical wedge is located adjacent to a transmitter side (e.g., adjacent to the first transceiver 210 or the second transceiver 220 that serves as the transmitter), the optical wedge may be located as a planar optical wedge to allow the optical beam to pass through without changing its direction. As another example, when the optical wedge is located on a receiver side (e.g., adjacent to the first transceiver 210 and the second transceiver 220 that serves as the receiver), the optical wedge may be set as a concentrating optical wedge to bring the optical beams together to ensure that the receiver receives a complete optical beam.

In some embodiments, the optical path adjustment module includes at least one adjustment unit. For example, each of the first transceiver 210 and/or the second transceiver 220 includes an adjustment unit.

In some embodiments, the at least one adjustment unit located adjacent to the receiver side is configured to aggregate signals received at the receiver, so that the receiver receives as much as possible a complete signal or a majority of the signal. For example, when the first transceiver 210 serves as the receiver, the adjustment unit may be located in a pathway in which the second transceiver 220 performs signal transmission to the first transceiver 210. As another example, when the second transceiver 220 serves as the receiver, the adjustment unit may be located at a pathway in which the first transceiver 210 performs signal transmission to the second transceiver 220.

By arranging that the first transceiver 210 and the second transceiver 220 on the rotor 211 and the rotational axis of the rotor 211 of the medical device 110 respectively, data are transmitted in the form of optical waves or electromagnetic waves. Thus, the data transmission distance between the medical device and the external device are increased, the data transmission bandwidth is increased, and the data transmission rate is improved. In addition, the bidirectional wireless data transmission is achieved, multiple data transmission paths in the medical device are avoided, thereby reducing the cost of the data transmission link of the medical device, and at the same time improving the efficiency of bidirectional data transmission.

In some embodiments, the at least one adjustment unit may be close to the first transceiver 210. For example, the at least one adjustment unit may be located in the first transceiver 210.

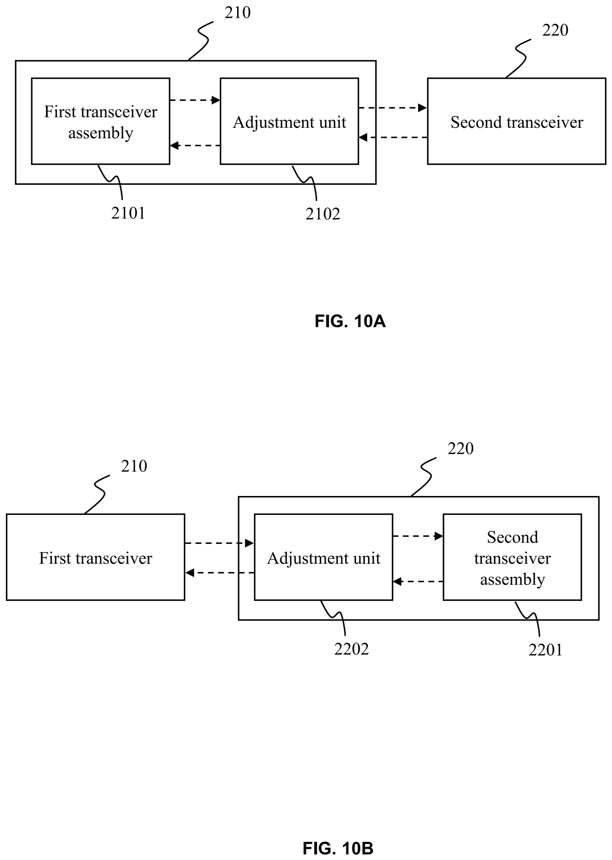

FIG. 10A is a schematic diagram of an exemplary arrangement of at least one adjustment unit according to some embodiments of the present disclosure.

As shown in FIG. 10A, the first transceiver 210 includes a first transceiver assembly 2101 and an adjustment unit 2102.

The adjustment unit 2102 in the first transceiver 210 is located in a signal transmission path of the first transceiver assembly 2101. For example, the adjustment unit 2102 is located between the first transceiver assembly 2101 and the second transceiver 220.

In some embodiments, the position of the adjustment unit 2102 may be fixed or adaptively adjusted. For example, during rotation of the rotor 211, both the first transceiver assembly 2101 and the adjustment unit 2102 rotate with the rotor 211, and the adjustment unit 2102 may be stationary or adjusted in real-time relative to the first transceiver assembly 2101. Thus, after the adjustment unit 2102 adjusts the direction of the first signal sent by the first transceiver assembly 2101, the second transceiver 220 is able to receive the first signal (and/or, after the adjustment of the direction of the second signal sent by the second transceiver 220, the first transceiver assembly 2101 is able to receive the second signal).

In some embodiments, the adjustment unit may be adjacent to the second transceiver 220. For example, the adjustment unit may be located in the second transceiver 220.

FIG. 10B is a schematic diagram of an exemplary arrangement of at least one adjustment unit according to some other embodiments of the present disclosure.

As shown in FIG. 10B, the second transceiver 220 may include a second transceiver assembly 2201 and an adjustment unit 2202.

The adjustment unit 2202 in the second transceiver 220 is located in a signal transmission path of the second transceiver assembly 2201. For example, the adjustment unit 2202 is located between the first transceiver 210 and the second transceiver assembly 2201.

In some embodiments, the position of the adjustment unit 2202 with respect to the second transceiver assembly 2201 may be fixed or flexibly adjustable. For example, during rotation of the rotor 211, the first transceiver 210 rotates with the rotor 211. In order to project signals emitted by the first transceiver 210 into the second transceiver assembly 2201, the adjustment unit 2202 may adjust the direction of the first signal sent by the first transceiver 210 during the rotation of the rotor 211, so that the second transceiver assembly 2201 receives the first signal sent by the first transceiver 210. The adjustment unit 2202 may also adjust the direction of the second signal sent by the second transceiver assembly 2201 during the rotation, so that the first transceiver 210 can receive the second signal sent by the second transceiver assembly 2201.

In some embodiments, the direction of the first signal and/or the second signal may be adjusted by setting the adjustment unit on the signal transmission path of the first transceiver assembly, and/or setting the adjustment unit on the signal transmission path of the second transceiver assembly, so that the signal path of the first transceiver 210 on the rotor 211 always points to the second transceiver 220 on the external stator, and the signal path of the second transceiver 220 on the stator always points to the first transceiver 210 on the rotor 211. Thus, the second transceiver 220 can receive the first signal transmitted by the first transceiver 210, and that the first transceiver 210 can receive the second signal transmitted by the second transceiver 220, thereby ensuring the bidirectional reliability and accuracy of the signal transmission.

In some embodiments, the optical path adjustment module may include the first adjustment unit 2102 and the second adjustment unit 2202. The first adjustment unit is located adjacent to the first transceiver 210 and the second transceiver 220 that serves as a receiver for adjusting the signal received by the receiver, and the second adjustment unit is located adjacent to the first transceiver 210 and the second transceiver 220 that serves as a transmitter for adjusting the signal sent from the transmitter.

In some embodiments, the first adjustment unit may include at least one of a second adjustment subunit located in the first transceiver 210 and a fourth adjustment subunit located in the second transceiver 220. The second adjustment unit may include at least one of a first adjustment subunit in the first transceiver 210 and a third adjustment subunit in the second transceiver 220.

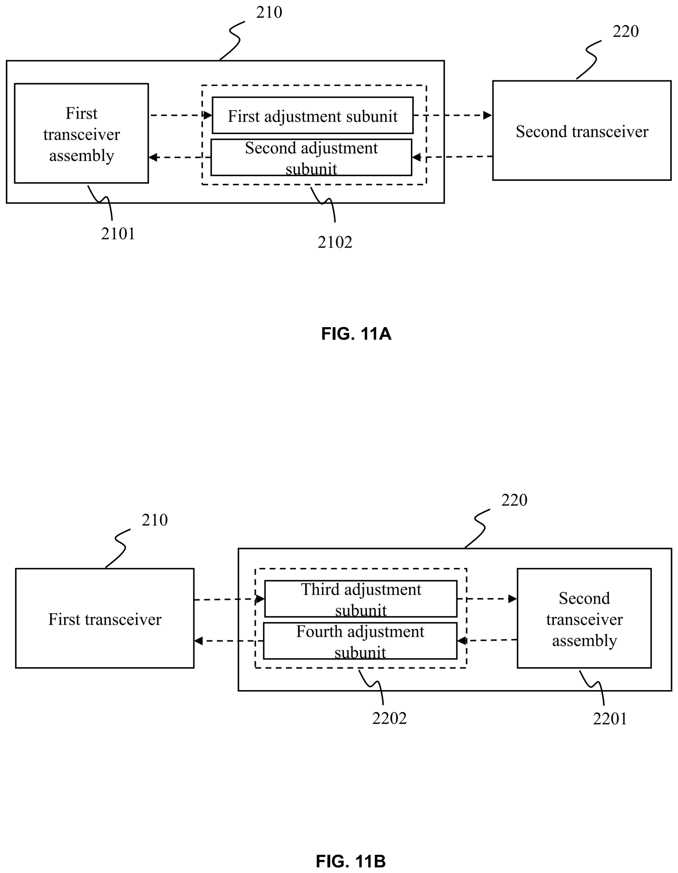

FIG. 11A is a schematic diagram of an exemplary arrangement of an adjustment unit according to some embodiments of the present disclosure.

As shown in FIG. 11A, in some embodiments, the adjustment unit 2102 may further include a first adjustment subunit and a second adjustment subunit. The first adjustment subunit is located at a signal transmitting path of the first transceiver assembly, and the second adjustment subunit is located at a signal receiving path of the first transceiver assembly. The first adjustment subunit is configured to adjust the direction of the first signal sent by the first transceiver assembly 2101. The second adjustment subunit is configured to adjust the direction of the first signal sent by the second transceiver 220.

In other words, on the rotor, different adjustment units are located at the sending path of the scanning data and the receiving path of the control signal. When the first transceiver assembly 2101 transmits a first signal, the first signal is transmitted to the second transceiver 220 after being directionally adjusted by the first adjustment subunit on the signal transmitting path. When the first transceiver assembly 2101 receives a second signal, the second signal transmitted by the second transceiver 220 is transmitted to the first transceiver assembly 2101 in the first transceiver 210 after being directionally adjusted by the second adjustment subunit on the signal receiving path in the first transceiver 210.

The adjustment of the signal direction by the adjustment unit may include changing a direction of transmission of the signal, or not changing a direction of transmission of the signal. The adjustment unit may make adjustments to the direction of the input signals so that the output signals are in a different direction from the input signals. Alternatively, the adjustment unit may not make adjustments to the direction of the input signals, maintaining the direction of transmission of the input signals so that the output signals are in the same direction as the input signals.

In some embodiments, the first adjustment subunit may not change the transmission direction of the first signal, so that the first signal from the first transceiver assembly 2101 is directly transmitted to the second transceiver 220 after passing through the first adjustment subunit. The second adjustment subunit may change the transmission direction of the second signal, so that the second signal from the second transceiver 220 converges to the first transceiver assembly 2101 after passing through the second adjustment subunit, so that the first transceiver assembly 2101 can accurately and completely receive the second signal.

In some embodiments, the first adjustment subunit may be a planar optical wedge, which does not change the direction of the first signal. The second adjustment subunit may be a concentrating optical wedge, which converges the second signal so that the first transceiver assembly 2101 receives a complete second signal.

FIG. 11B is a schematic diagram of an exemplary arrangement of an adjustment unit according to some other embodiments of the present disclosure.

As shown in FIG. 11B, the adjustment unit 2202 may further include a third adjustment subunit and a fourth adjustment subunit. The third adjustment subunit is located at a signal receiving path of the second transceiver assembly 2201. The fourth adjustment subunit is located at the signal transmitting path of the second transceiver assembly 2201. The third adjustment subunit is configured to adjust the direction of the first signal, and the fourth adjustment subunit is configured to adjust the direction of the second signal.

In other words, on the side of the stator, for the receiving path of the scanning data and the transmitting path of the control signals, respectively, different adjustment units are located. When the second transceiver assembly 2201 receives the first signal, the first signal sent by the first transceiver 210 is transmitted to the second transceiver assembly 2201 in the second transceiver 220 after being directionally adjusted by the third adjustment subunit in the signal receiving path in the second transceiver 220. When the second signal is sent by the second transceiver assembly 2201, the second signal is transmitted to the first transceiver 210 after being directionally adjusted by the fourth adjustment subunit in the signal transmitting path.

The adjustment of the signal direction by the adjustment unit may include changing the direction of transmission of the signal, or not changing the direction of transmission of the signal. Exemplarily, the third adjustment subunit may change the transmission direction of the first signal such that the first signal from the first transceiver assembly 2101 is converged to the second transceiver assembly 2201 after it passes through the third adjustment subunit, so that the second transceiver assembly 2201 can accurately receive the first signal. The fourth adjustment subunit may not change the transmission direction of the second signal, so that the second signal from the second transceiver 220 is transmitted to the first transceiver 210 after passing through the fourth adjustment subunit.

In some embodiments, the third adjustment subunit may be a concentrating optical wedge. For example, the first signal is converged, such that the second transceiver assembly 2201 receives the complete first signal. The fourth adjustment subunit may be a planar optical wedge. For example, the direction of the second signal is not changed.

In some embodiments, the first transceiver 210 may include the first transceiver assembly 2101 and the adjustment unit 2102, and the second transceiver 220 may include the second transceiver assembly 2201 and the adjustment unit 2202. The adjustment unit 2102 is located between the first transceiver assembly 2101 and the adjustment unit 2202, and is located at a side adjacent to the first transceiver 2101. The adjustment unit 2202 is located between the adjustment unit 2102 and the second transceiver 2201, and is located at a side adjacent to the second transceiver 2201.

The adjustment unit 2102 may be configured to adjust the direction of the first signal and/or the second signal. The adjustment unit 2202 may be configured to adjust the direction of the first signal and/or the second signal.

In some embodiments, the adjustment unit 2102 may be configured to adjust the direction of the second signal sent by the second transceiver assembly 2201, and the adjustment unit 2202 may be configured to adjust the direction of the first signal sent by the first transceiver assembly 2101. That is to say, when the first transceiver assembly 2101 sends the first signal to the second transceiver assembly 2201, the adjustment unit 2102 does not change the direction of the first signal, and the direction of the first signal is adjusted and then transmitted to the second transceiver assembly 2201, so that the second transceiver assembly 2201 can receive the first signal. When the second transceiver assembly 2201 sends the second signal to the first transceiver assembly 2101, the adjustment unit 2202 does not change the direction of the second signal, and transmits to the first transceiver assembly 2101 after adjusting the direction of the second signal through the adjustment unit 2102, so that the first transceiver assembly 2201 can receive the first signal.

Exemplarily, the adjustment unit 2102 may include a first adjustment subunit located in the signal transmission path and a second adjustment subunit located in the signal receiving path. The adjustment unit 2202 may include a third adjustment subunit located in the signal receiving path and a fourth adjustment subunit located in the signal receiving path. The first adjustment subunit may not adjust the direction of the first signal sent by the first transceiver assembly 2101, and the direction of the first signal sent by the first transceiver assembly 2101 may be adjusted by the third adjustment subunit, thereby enabling the second transceiver assembly 2201 to completely and accurately receive the first signal sent by the first transceiver assembly 2101. The fourth adjustment subunit may not adjust the direction of the second signal sent by the second transceiver assembly 2201, and the direction of the second signal sent by the second transceiver assembly 2201 is adjusted by the second adjustment subunit, so that the first transceiver assembly 2101 can completely and accurately receive the second signal sent by the second transceiver assembly 2201.

According to arranging the first adjustment subunit and the second adjustment subunit in the first transceiver 210 on the rotor 211 and/or the third adjustment subunit and the fourth adjustment subunit in the second transceiver 220 on the stator 221 of the transmission system, the reliability and accuracy of the bidirectional signal transmission may be improved.

In data transmission based on optical waves, the first transceiver 210 may be a first optical beam transceiver, and the second transceiver 220 may be a second optical beam transceiver. In the case where the first transceiver 210 includes the first transceiver assembly 2101 and the adjustment unit 2102, the first transceiver assembly 2101 may be a first optical beam transceiver, and the adjustment unit 2102 may be a first optical wedge.

In the case of data transmission based on electromagnetic waves, the electromagnetic waves include, but are not limited to, terahertz, millimeter waves, or the like. In the case where the electromagnetic waves are terahertz, the first transceiver 210 may be a first terahertz transceiver, the second transceiver 220 may be a second terahertz transceiver. **In** the case where the first transceiver 210 includes the first transceiver assembly 2101 and the adjustment unit 2102, the first transceiver assembly 2101 may be a first terahertz transceiver, and the adjustment unit 2102 may be a first terahertz steering.

**In** the case where the electromagnetic wave is a millimeter wave, the first transceiver 210 may be a first millimeter wave transceiver, and the second transceiver 220 may be a second millimeter wave transceiver. In the case where the first transceiver 210 includes the first transceiver assembly 2101 and the adjustment unit 2102, the first transceiver assembly 2101 may be a first millimeter wave transceiver, and the adjustment unit 2102 may be a rotatable first millimeter wave transceiver antenna.

In the case of data transmission via optical waves, the first transceiver 210 may be a first optical beam transceiver, and the second transceiver 220 may be a second optical beam transceiver. In the case that the second transceiver 220 includes the second transceiver assembly 2201 and the adjustment unit 2202, the second transceiver assembly 2201 may be a second optical beam transceiver, and the adjustment unit 2202 may be a second optical wedge.

In the case of data transmission based on electromagnetic waves and the electromagnetic waves are terahertz, the first transceiver 210 may be a first terahertz transceiver, and the second transceiver 220 may be a second terahertz transceiver. In the case where the second transceiver 220 includes the second transceiver assembly 2201 and the adjustment unit 2202, the second transceiver assembly 2201 may be a second terahertz transceiver, and the adjustment unit 2202 is a second terahertz steering.

In the case where the data transmission is based on electromagnetic waves and the electromagnetic waves are millimeter waves, the first transceiver 210 may be a first millimeter wave transceiver, and the second transceiver 220 may be a second millimeter wave transceiver. In the case where the second transceiver 220 includes the second transceiver assembly 2201 and the adjustment unit 2202, the second transceiver assembly 2201 may be a second millimeter wave transceiver, and the adjustment unit 2202 may be a rotatable second millimeter wave transceiver antenna.

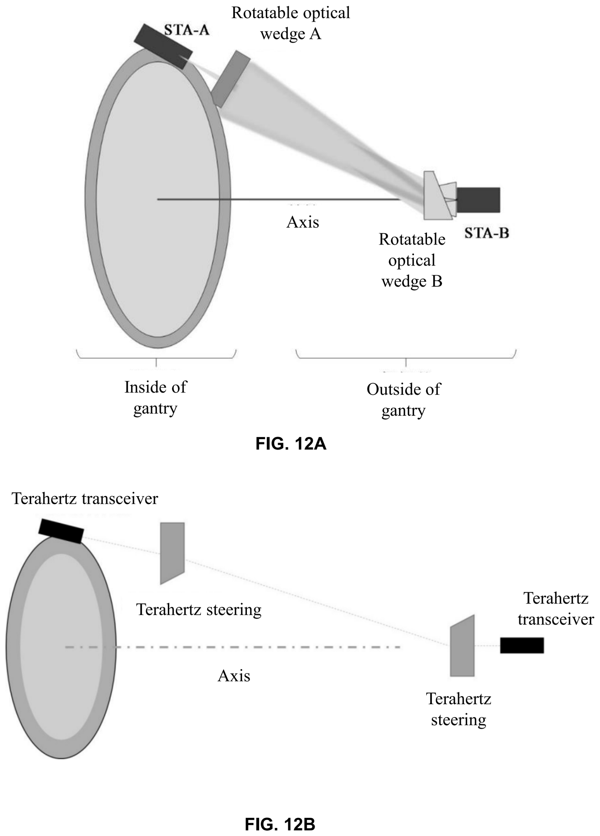

FIG. 12A is a schematic diagram of data transmission based on optical waves according to some embodiments of the present disclosure.

As shown in FIG. 12A, data of the transmission system 200 is transmitted via optical waves, the first transceiver 210 (including an optical beam transceiver STA-A) is arranged on the rotor 211 inside the gantry of the medical device, and the second transceiver 220 (including an optical beam transceiver STA-B) is arranged outside the gantry and located at a rotational axis of the gantry. Furthermore, on the optical beam transmission path, a rotatable optical wedge A is located at a side of the first transceiver 210 that is adjacent to the second transceiver 220 and the rotatable optical wedge A is located at the rotor 211. A rotatable optical wedge B is located at a side of the second transceiver 220 that is adjacent to the first transceiver 210 and the rotatable optical wedge B is located at the stator 221.

After the first transceiver 210 in the rotor performs electrical/optical conversion of the received scanning data, the first optical beam is sent out by the optical beam transceiver STA-A in the first transceiver 210, and the first optical beam is converged in a signal receiving range of the optical beam transceiver STA-B in the second transceiver 220 after passing through the rotatable optical wedge A and the rotatable optical wedge B, so that the optical beam transceiver STA-B in the second transceiver 220 receives the first optical beam, thereby obtaining the scanning data after being parsed .

After the second transceiver 220 on the outer stator of the gantry performs electrical/optical conversion of the received control signal, the second optical beam is sent out by the optical beam transceiver STA-B in the second transceiver 220, and the second optical beam is converged in the signal receiving range of the optical beam transceiver STA-A in the first transceiver 210 after passing through the rotatable optical wedge B and the rotatable optical wedge A, so that the optical beam transceiver STA-A in the first transceiver 210 receives the second optical beam, thereby obtaining control signal after being parsed.

For the first optical beam, the rotatable optical wedge A may converge the first optical beam or may not converge the first optical beam, and the rotatable optical wedge B may converge the optical path of the first optical beam, so that the first optical beam may be converged at the optical beam transceiver STA-B of the second transceiver 220. For the second optical beam, the rotatable optical wedge B may converge the second optical beam or may not converge the second optical beam, and the rotatable optical wedge A may converge on the optical path of the second optical beam so that the second optical beam can be converged on the optical beam transceiver STA-A of the first transceiver 210.

In some embodiments, when the transmission system 200 transmits based on the electromagnetic waves, the electromagnetic waves may be terahertz or millimeter waves.

FIG. 12B is a schematic diagram of data transmission based on terahertz according to some embodiments of the present disclosure.

As shown in FIG. 12B, the data is transmitted via terahertz, the transmission system 200 may include a terahertz transceiver and a terahertz steering on the rotor within the gantry, and a terahertz steering and a terahertz transceiver on the stator outside the gantry. The process of data transmission is similar to data transmission based on optical waves, as may be seen in the preceding related description.

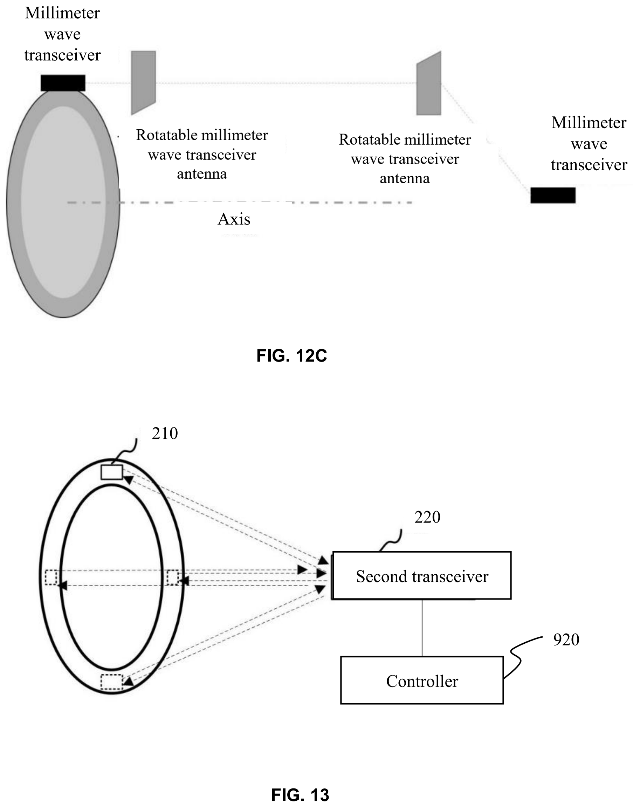

FIG. 12C is a schematic diagram of data transmission based on millimeter waves according to some embodiments of the present disclosure.

As shown in FIG. 12C, the data is transmitted via millimeter wave, the transmission system 200 may include a millimeter wave transceiver and a rotatable millimeter wave transceiver antenna on a rotor within the gantry, and a rotatable millimeter wave transceiver antenna and a rotatable millimeter wave transceiver antenna on a stator outside the gantry. The process of data transmission is similar to data transmission based on optical waves, as may be seen in the preceding related description.

FIG. 13 is a schematic diagram of a transmission system of a medical device according to some other embodiments of the present disclosure.

As shown in FIG. 13, the transmission system 200 may further include a controller 920.

The controller 920 is connected to the second transceiver 220. In some embodiments, the controller is configured to adjust a signal reception range of the second transceiver 220, and/or to control the optical path adjustment module 910. The signal reception range of the second transceiver 220 refers to a maximum angle of optical that the second transceiver 220 can receive.

In some embodiments, in response to determining that the power of the first signal is less than or equal to a preset power threshold, the controller 920 adjusts the signal receiving range of the second transceiver 220.

Exemplarily, the controller 920 may be located at the same location as the second transceiver 220 or at a different location, and the controller 920 may be connected to the second transceiver 220 via wired, wireless, etc., to realize data transmission. For example, the controller 920 may obtain the power of the first signal by detecting the first signal passing through the adjustment unit 2202 in the second transceiver 220. For example, the controller 920 may also obtain the power of the first signal by detecting the first signal acquired by the second transceiver assembly 2201 in the second transceiver 220. For example, the controller 920 may also obtain the power of the first signal by detecting the first signal acquired by the adjustment unit 2202. The manner of detecting the power of the first signal may be performed according to the actual situation.

In some embodiments, in response to determining that the power of the first signal is less than or equal to a preset power threshold, the controller 920 may adjust the signal reception range of the second transceiver 220. Exemplarily, the controller 920 may send an adjustment instruction to the second transceiver 220 to instruct the second transceiver 220 to adjust the signal reception range. The controller 920 may also directly control the second transceiver 220 and adjust the signal reception range of the second transceiver 220.

The preset power threshold may be related to a rated conversion power of the second transceiver 220. The rated conversion power of the second transceiver 220 refers to a minimum power by which the second transceiver 220 can recover complete scanning data based on the received first signal. In response to determining that the power of the first signal received by the second transceiver 220 is greater than or equal to the rated conversion power, the second transceiver 220 may recover complete scanning data based on the received first signal. Conversely, in response to determining that the power of the first signal received by the second transceiver 220 is less than the rated conversion power, the complete scanning data cannot be recovered, resulting in data loss. The preset power threshold may be equal to the rated conversion power or may be greater than the rated conversion power. In addition, the rated conversion power of the second transceiver 220, is related to a data transmission bandwidth, and the data transmission bandwidth is directly proportional to the rated conversion power of the second transceiver 220. Thus, in practical application, the second transceiver 220 that satisfies the demand for the data transmission bandwidth may be selected according to a desired data transmission bandwidth for the transmission of the scanning data.

Exemplarily, in practical application, if there is no second transceiver 220 that meets the demand of the data transmission bandwidth, a great bandwidth for the data transmission may be achieved by increasing a transmit power of the first transceiver 210, or by lowering a folding rate of the signal transmission. Alternatively, when the data transmission bandwidth is great, a pulse frequency of a photoelectric converter in the first transceiver 210 is great, corresponding data transmission rate is fast.

Exemplarily, the controller 920 may adjust the signal reception range of the second transceiver 220 by adjusting the adjustment unit 202 in the second transceiver 220. For example, the position and/or direction of the adjustment unit 202 may be adjusted to increase the signal receiving range of the adjustment unit 202 to increase the signal power of the first signal received by the second transceiver 220 201.

In some embodiments, in adjusting the position and/or the direction of the adjustment unit 2202 in the optical path adjustment module, the adjustment unit 202 may be located at an adjustment platform (e.g., a pan tilt). The controller 920 may control the adjustment module to move according to a preset adjustment strategy to change the position and/or direction of the adjustment unit 202. The preset adjustment strategy may include at least one of a positional movement direction, a positional movement distance, a rotation direction, and a rotation angle.

It is noted that the controller 920 may adjust the adjustment unit 202 at least once according to the preset adjustment strategy. After each adjustment, the controller 920 may re-detect the power of the adjusted first signal until the power of the first signal is greater than or equal to the preset power threshold.

In some embodiments, before the medical device leaves the factory or before an image scanning process is performed, a pre-test may be performed to detect whether the power of the first signal received by the second transceiver 220 satisfies the power demand (e.g., whether the power of the first signal is greater than or equal to the preset power threshold). If the power demand is satisfied, the image scanning process may be carried out normally. If the power demand is not satisfied, the power of the first signal received by the second transceiver 220 may be increased by adjusting the signal receiving range of the second transceiver 220 to ensure stable transmission of the scanning data.

In some embodiments, during the process of image scanning process, the power of the first signal received by the second transceiver 220 may also be detected in real time. In response to detecting that the power of the first signal received by the second transceiver 220 is less than the preset power threshold, the second transceiver 220 is considered to be no longer able to completely receive the scanning data sent by the first transceiver 210, or the scanning data received by the second transceiver 220 is incomplete. In this case, the current image scanning process may be stopped, and the second transceiver 220 may be adjusted and corrected to ensure that the power of the first signal received by the second transceiver 220 meets the power demand, and then the image scanning process may be performed again. Thus, it is possible to avoid that the patient finding that the image scanning data is missing after a complete image scanning process, which causes the patient to receive excessive radiation effects. In addition, if during the scanning process, the scanning data received by the stator terminal is found to be incomplete and the current scanning process stops in time, the second transceiver 220 on the stator can also be adjusted or corrected at the first time, and the image scanning process may be performed again after the second transceiver 220 being adjusted, thus shortening the time duration of the entire image scanning process and improving the efficiency of the image scanning process.

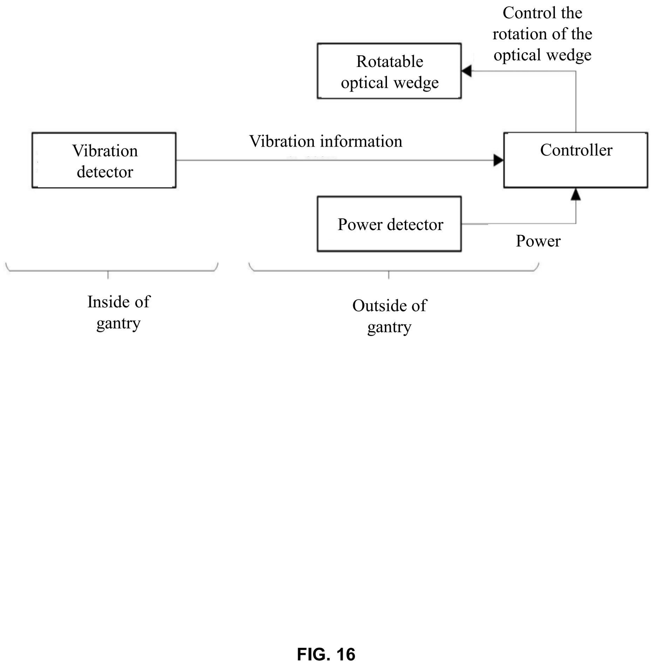

In some embodiments, the controller 920 may control the optical path adjustment module 910. For example, adjusting a rotatable optical wedge in the optical path adjustment module 910 based on vibration information of the rotor. More detailed description may be found in FIG. 16 of this disclosure and its related description.

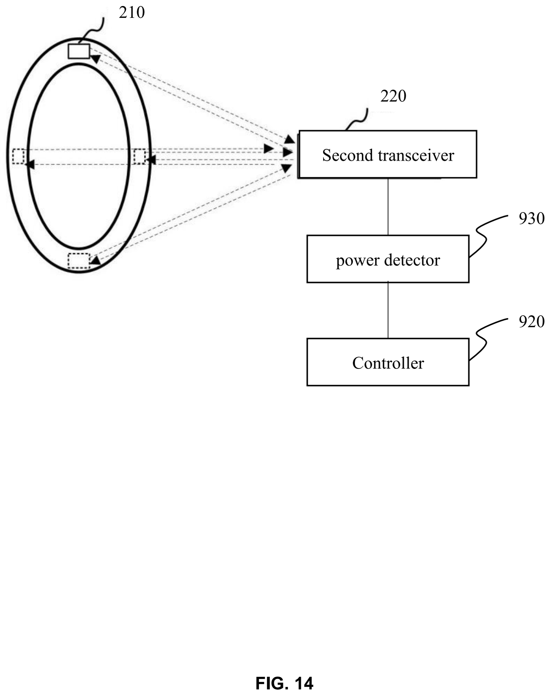

FIG. 14 is a schematic diagram of a transmission system of a medical device according to some other embodiment of the present disclosure.

As shown in FIG. 14, the transmission system 200 may also include a power detector 930.

The power detector 940 is configured to detect a power of an optical beam received by the first transceiver 210 or the second transceiver 220.

In some embodiments, the controller 930 may control the optical path adjustment module based on the power of the optical beam received by the first transceiver 210 or the second transceiver 220.

The power detector 940 is connected to the second transceiver 220 and the controller 920, respectively. The power detector 940 may be configured to detect the power of the first signal received by the second transceiver 220 and send the power to the controller 920.

Exemplarily, the controller 920 may detect the power of the first signal by detecting the power of the first signal received by the second transceiver 220 by the power detector 940 to obtain the power of the first signal, such that the controller 920 may obtain the power of the first signal from the power detector 940. The power of the first signal may include a power value of the first signal received by the second transceiver 220 at at least one moment in time, and thereby, the controller 920 may make a judgment on a magnitude relationship between the power value of the first signal and a preset power threshold. Thus, in response to determining that the power of the first signal is less than the preset power threshold, the signal reception range of the second transceiver 220 is adjusted.

Exemplarily, in the case where the second transceiver 220 includes the second transceiver assembly and the adjustment unit, the power detector 940 may be connected to the second transceiver assembly for detecting the power of the first signal received by the second transceiver assembly. Or, the power detector 940 may also detect the power of the first signal received by the adjustment unit, thereby obtaining the power of the first signal.

It should be noted that the power detector 940 may include, but is not limited to, a power detector, a power detecting module, or the like of any form or any structure, and the type and structure of the power detector 940 may be set according to the actual circumstances or demand setting.

Exemplarily, in the case where the power of the first signal includes the power value of the first signal at a plurality of moments, the way in which the controller 920 adjusts the signal reception range of the second transceiver 220 may include: after the controller 920 determines that the power value of the first signal is less than a preset power threshold at a current moment, adjusting the signal reception range of the second transceiver 220. Alternatively, in the case where the controller 920 determines that the power values of the first signal are all less than the preset power threshold at a preset count of consecutive moments, adjusting the signal reception range of the second transceiver 220. Adjusting the signal receiving range of the second transceiver 220 may include adjusting a position and/or an orientation of an adjustment unit in the second transceiver 220 to adjust the signal receiving range of the adjustment unit so as to increase the signal receiving range of the second transceiver 220.

Exemplarily, in the case where the adjustment unit is an optical wedge, the position and/or direction of the optical wedge may be adjusted so that more of the first (optical beam) signal may be irradiated within a center region of the optical wedge to improve a clustering effect of the first (optical beam) signal, thereby improving the signal power of the first (optical beam) signal received by the second transceiver assembly.

Exemplarily, in response to determining that the power of the first signal is less than the preset power threshold during the image scanning process, an optional realization may quickly adjust the signal receiving range of the second transceiver 220 without stopping the medical device (to continue with the current scanning process). In response to determining that the power of the first signal is greater than or equal to the preset power threshold after the adjustment, the subsequent scanning process and the data transmission process may be continued. **In** response to determining that the power of the first signal after being adjusted is still less than the preset power threshold, then the medical device may be stopped for checking and correcting the transmission system. In some embodiments, quickly adjusting the signal receiving range of the second transceiver 220 may include adjusting the signal receiving range of the second transceiver 220 at least once, using a preset adjustment strategy.

Exemplarily, during the image scanning process, if a situation occurs in which the power of the first signal is less than a preset power threshold, it is also possible to immediately abort the current scanning process, a shutdown inspection is carried out, and the transmission system is corrected, and when the transmission system returns to normal, the image scanning process may be restarted.

In some embodiments, the power detector is located at the stator to detect the power of the first signal received by the second transceiver 220 in order to perform a real-time monitoring of the reliability and stability of the data transmission in the image scanning process. In the event of a data transmission abnormality, the transmission system may be immediately suspended, which not only improves the reliability of the data transmission, but also avoids the patient from being affected by more radiation, and the efficiency of the impact scanning may be improved.

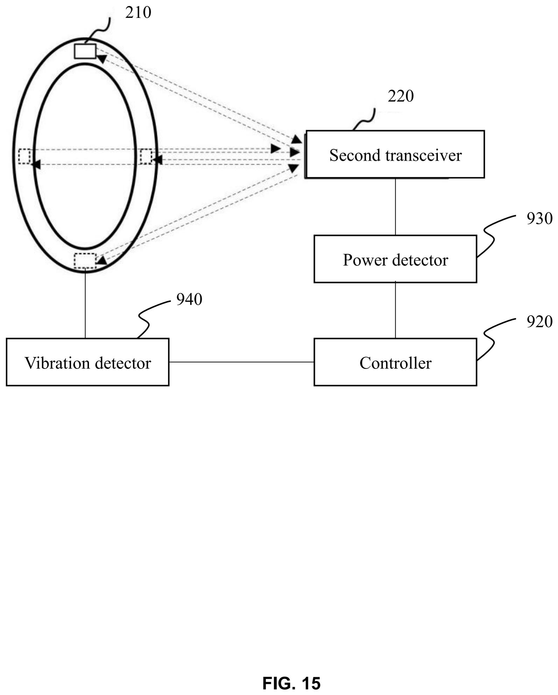

FIG. 15 is a schematic diagram of a transmission system of a medical device according to some other embodiment of the present disclosure.

As shown in FIG. 15, the transmission system 200 may further include a vibration detector 940.

The vibration detector 940 is configured to detect vibration information of a rotor 211 and transmit the vibration information to a controller.

In some embodiments, the controller may control an optical path adjustment module based on the vibration information sent by the vibration detector. For example, the controller may adjust the signal reception range of a second transceiver 220 based on the vibration information.

In some embodiments, the vibration detector 940 is connected to the rotor 211 and the controller 920, respectively.

The vibration information is data reflecting the vibration of a first transceiver 210.

The first transceiver 210 is located at the rotor of the medical device, so that the first transceiver 210 may follow the rotor to rotate together. In the process of rotating, the gantry will inevitably be jittered, resulting in the first transceiver 210 being jittered as well. In the case of jittering of the first transceiver 210, the first (optical beam) signal emitted from the first transceiver 210 may not be able to accurately irradiate in the optical beam receiving range of the second transceiver 220 (e.g., the center region of the optical wedge), resulting in fluctuations in the power of the first (optical beam) signal received by the first transceiver 210. Fluctuations in the power of the first (optical beam) signal not only cause unstable data transmission, but also may cause a situation where the power is less than the preset power threshold, leading to incomplete data parsing and thus serious problems of data loss.

In some embodiments of the present disclosure, the vibration detector 50 is located at the rotor 211 so that the controller 920 at the stator 221 can make real-time adjustments to the signal receiving range of the second transceiver 220 at the stator based on the vibration information at the rotor 211, so that the second transceiver 220 can follow the first transceiver 210 to carry out the same vibration to realize vibration elimination between the second transceiver 220 and the first transceiver 210 (e.g., to realize relative stationarity between the second transceiver 220 and the first transceiver 210), thereby always ensuring that the first (optical beam) signal emitted by the first transceiver 210 may be accurately irradiated within the signal receiving range of the second transceiver 220, so as to ensure that the power of the first (optical beam) signal received by the second transceiver 220 can satisfy a certain requirement of stability, thereby improving the stability and reliability of data transmission.

In some embodiments, in the case where the second transceiver 220 includes a second transceiver assembly and an adjustment unit, the adjustment unit may be located at an adjustment platform (e.g., a pan tilt), and the controller 920 may make real-time adjustments to the adjustment platform where the adjustment unit is located based on the received vibration information of the rotor 211 to realize vibration elimination. In some embodiments, since the first transceiver 210 is located at the rotor 211, the vibration information of the rotor 211 may be configured to characterize the vibration information of the first transceiver 210. Furthermore, the collection of the vibration information of the rotor 211, instead of the vibration information of the first transceiver 210, the vibration collecting device may be fixedly and statically mounted at a preset position on the rotor 211, without having to follow the rotor 211 to rotate, which can effectively avoid the influence of the vibration that occurs during the rotation process, and improve the accuracy of the vibration information to improve the accuracy of the control of the vibration elimination, which can in turn improve the stability of the data transmission.

In some embodiments of the present disclosure, by setting the vibration detector on the rotor 211, collecting the vibration information of the first transceiver 210 in the rotation process, and then adjusting the signal reception range of the second transceiver 220 based on the vibration information, the first signal from the first transceiver 210 can always be irradiated within the effective signal receiving range of the second transceiver 220, realizing vibration elimination, thereby ensuring the stability and reliability of the first signal received by the second transceiver 220, and improving the efficiency of data transmission.

FIG. 16 is a schematic diagram of controlling an optical path adjustment module according to some embodiments of the present disclosure.

As shown in FIG. 16, since the rotor 211 generates vibration during the rotating process, an optical path cannot be ideally converged at one point, so the vibration detector may be located at the stator 221 inside the gantry to collect the vibration information from the rotor 211 and real-time send the vibration information to the controller 920 outside the gantry, to make the controller 920 can adjust the rotatable optical wedge, such as the optical wedge B, according to the vibration information of the rotor 211, so as to realize the vibration elimination. The rotatable optical wedge B can re-converge the optical path that is offset due to the vibration, so that the optical path of the first optical beam sent from the first transceiver 210 on the rotor 211 in the scanning process is always converged in the optical beam receiver of the second receiver, to ensure the stability of data transmission.

In some embodiments, in order to ensure the reliability of the scanning data transmission, a power detector may also be located at the data receiving end outside the gantry to collect the power of the first optical beam received by the second optical transceiver. The controller 920, based on the power, may realize fine-tuning of the rotatable optical wedge B to ensure the stability of the received power at the receiving end, and thus ensure the stability of the transmission rate. In addition, through the detection of the received power, it is also possible to timely abort the scanning in the event of an abnormality or a malfunction of the data transmission system, and carry out repairs and corrections to the data transmission system immediately.

Because of the reversibility of the optical path, when one of the rotatable optical wedges is adjusted so that one way of the transmission of the optical beams meets the demands, the other way of the transmission of the optical beams will also meet the transmission demands. Therefore, one or two optical wedges may be adjusted according to the actual needs to make the optical beam meet the transmission demands. Exemplarily, in adjusting the optical wedge, only the rotatable optical wedge A on the rotor may be adjusted, or the rotatable optical wedge B on the stator outside of the gantry may be adjusted, or both the rotatable optical wedge A and the rotatable optical wedge B may be adjusted, which is not limited by this embodiment.

During the operation of the medical device, the rotor 211 rotates at a high speed, and a device for adjusting the signal transmission path, such as the optical path adjustment module in the transmission system 200, also needs to switch rapidly to adjust the angle of the optical wedge, the receiving range of the receiver, or the like. In some embodiments, an adjustment model may be pre-trained, and an input of the adjustment model includes the vibration information detected by the vibration detector, and an output includes an adjustment signal for at least one device in the transmission system 200.

In some embodiments, the foregoing adjustment model may be a machine learning model. With the machine learning model, the efficiency of determining the adjustment signals may be improved with the help of the empirical learning of the a priori data in order to adapt to the high-speed changes in the signal transmission path brought about by the high-speed rotation of the rotor 211, and to ensure efficiency of the data transmission of the transmission system 200.

In some embodiments, the stator is located outside the gantry of the medical device, and compared with the traditional carbon brush and slip ring manner and capacitive coupling manner, the transmitter on the rotor 211 may be utilized to transmit the data collected in the rotor 211 along a rotor face (axial direction) to the receiving end outside the gantry under the condition of not increasing the size of the existing gantry. For example, the transmitter is integrally located on an end face of the rotor 21, and the transmitting path is converged into a cone shape at one point during the rotation process of the rotor 211. Because the receiver of the stator 221 outside the gantry is not integrated into the gantry inside, the receiver may be effectively prevented from randomly vibrating with the gantry to improve the stability of the signal power of the signal received by the receiver. The adoption of the transmission system 200 can effectively improve the signal transmission distance, bandwidth and anti-jamming ability, and can avoid the aperture size of the rotor 211 limiting a processing size of the traditional slip ring antennas, so that the size of the aperture size of the rotor 211 no longer affects the bandwidth of the slip ring.

FIG. 17 is a schematic diagram of a transmission system of a medical device according to some other embodiments of the present disclosure.

In some embodiments, bidirectional transmission between the first transceiver 210 and the second transceiver 220 is a transmission via electromagnetic waves.

At this point, the first transceiver 210 may be located on the circumference of the rotor 211. The first transceiver 210 includes a first phased-array antenna 1710. The second transceiver 220 may include a second phased-array antenna 1720. The second phased-array antenna 1720 may be located within a coverage area of radio frequency beams emitted from the first phased-array antenna 1710. Additionally or alternatively, the first phased-array antenna 1710 is located within a coverage area of radio frequency beams emitted from the second phased-array antenna 1720.

In some embodiments, the second phased-array antenna 1720 may also be located outside the coverage area of the radio frequency beams emitted by the first phased-array antenna 1710. The first phased-array antenna 1710 may also be located outside the coverage area of the radio frequency beams emitted by the second phased-array antenna 1720. When a phased-array antenna is outside the radio frequency beams coverage area, signal transmission can be achieved through a repeater, for example, by transmitting the signal to the receiver via the repeater.

Further description of the first transceiver 210, the second transceiver 220, and the rotor may be found in the preceding related description.

A phased-array antenna (e.g., the first phased-array antenna 1710 or the second phased-array antenna 1720) is an array composed of a plurality of radiation units. The phased-array antenna is an antenna system that realizes rapid beam pointing or shaping through electronic control of phase differences of signals from each radiation unit. The phased-array antenna is configured for directional signal transmission and space-selective reception.

The first phased-array antenna 1710 is a phased-array antenna located at the first transceiver 210 to emit and receive radio frequency beams.

In some embodiments, the first phased-array antenna 1710 may include a phased-array transmitting element and a phased-array receiving antenna. When the first transceiver 210 serves as a transmitter, the phased-array transmitting element in the first phased-array antenna 1710 begins to operate, and when the first transceiver 210 serves as a receiver, the phased-array receiving antenna in the first phased-array antenna 1710 begins to operate.

The second phased-array antenna 1720 is a phased-array antenna located at the second transceiver 220 to emit and receive radio frequency beams.

In some embodiments, the second phased-array antenna 1720 may include a phased-array transmitting element and a phased-array receiving antenna. When the second transceiver 220 serves as a transmitter, the phased-array transmitting element in the second phased-array antenna 1720 begins to operate, and when the second transceiver 220 serves as a receiver, the phased-array receiving antenna in the second phased-array antenna 1720 begins to operate.

As shown in FIG. 17, the transmission system 200 may include the first phased-array antenna 1710 and the second phased-array antenna 1720 when the bidirectional transmission is a transmission via electromagnetic waves. The first phased-array antenna 1710 is located at the rotor 211 of the medical device 110. The second phased-array antenna 1720 is located at the stator 221 (e.g., on a separate support frame 1750 outside the gantry) of the medical device 110. The second phased-array antenna 1720 is located within the coverage area of radio frequency beams emitted by the first phased-array antenna 1710. In some embodiments, the radio frequency beams of the first phased-array antenna 1710 are emitted in a direction from the circumference of the rotor 211 towards the second phased-array antenna 1720.

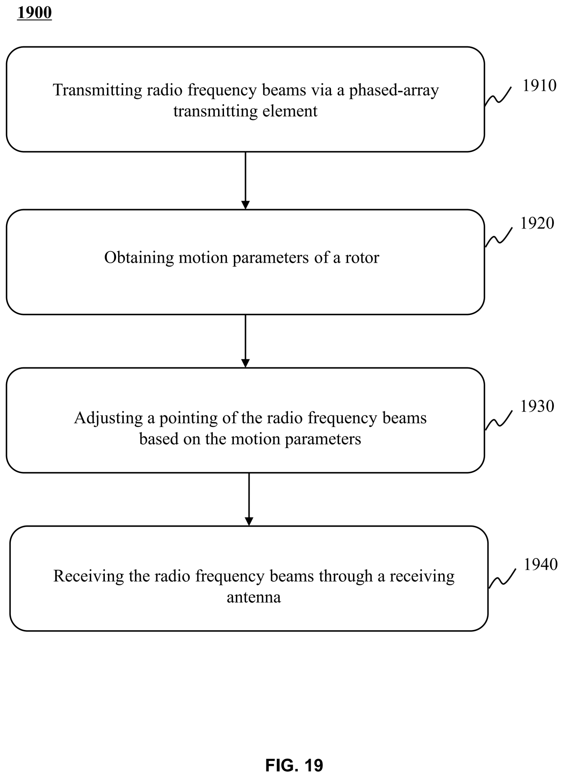

In some embodiments, the transmission system 200 may also include a synchronization module 1740. More description of the synchronization module 1740 may be found in FIG. 19 and its related description.

During the scanning process of the medical device 110, the phased-array transmitting element in the first phased-array antenna 1710 transmits the signals generated by the interaction of the rays with the target object in the form of the radio frequency beams for data transmission, and the phased-array receiving antenna in the second phased-array antenna 1720 receives the aforementioned radio frequency beams, thereby realizing data transmission of the signal generated by the interaction of the rays with the target object, and realizing subsequent image reconstruction based on the transmitted data.

The first phased-array antenna 1710 is located at the rotor 211 of the medical device 110 and follows the rotation of the rotor 211. The radio frequency beams emitted by the phased-array transmitting element of the first phased-array antenna 1710 contain signals (e.g., scanning data) generated by the interaction of the rays with the target object. The position of the phased-array receiving antenna of the second phased-array antenna 1720 is located in the coverage area of the radio frequency beams and is capable of receiving the radio frequency beams in real-time. By setting the position of the phased-array receiving antenna of the second phased-array antenna 1720 to be located in the coverage area of the radio frequency beams, the phased-array receiving antenna of the second phased-array antenna 1720 is able to follow the rotation of the phased-array transmitting element of the first phased-array antenna 1710 in real-time and receive the radio frequency beams. The transmission and reception of the radio frequency beam are realized by the first phased-array antenna 1710 and the second phased-array antenna 1720. The position of the second phased-array antenna 1720 may be arbitrarily placed within the coverage area of the radio frequency beams.

Similarly, the phased-array transmitting element of the second phased-array antenna 1720 may transmit the radio frequency beams carrying control signals. The phased-array receiving antenna of the first phased-array antenna 1710 is located in a coverage area of the radio frequency beams to receive the control signals transmitted by the phased-array transmitting element of the second phased-array antenna 1720. The second phased-array antenna 1720 is located at the stator 221 of the medical device 110.

In some embodiments, the first phased-array antenna 1710 or the second phased-array antenna 1720 includes a plurality of phased-array elements, and the plurality of phased-array elements work at different frequencies or at different time interval. The plurality of phased-array elements in the first phased-array antenna 1710 or the second phased-array antenna 1720 have independent operating frequencies or time intervals, which realizes frequency division multiplexing or time division multiplexing, avoiding mutual interference. The signals are reasonably divided and arranged in the frequency and time dimensions to improve the transmission efficiency and resource utilization between the first phased-array antenna 1710 and the second phased-array antenna 1720.

The phased-array elements are the basic transmitting or receiving unit that constitutes a phased-array antenna, and by independently regulating the phase and amplitude of an excitation signal, the beam forming and electronic scanning functions in the array are achieved. The phased-array elements may include a physical carrier (e.g., patch antenna, dipole, waveguide port, etc.) for electromagnetic wave radiation or reception.

In some embodiments, the phased-array elements include a phased-array transmitting element and a phased-array receiving element.

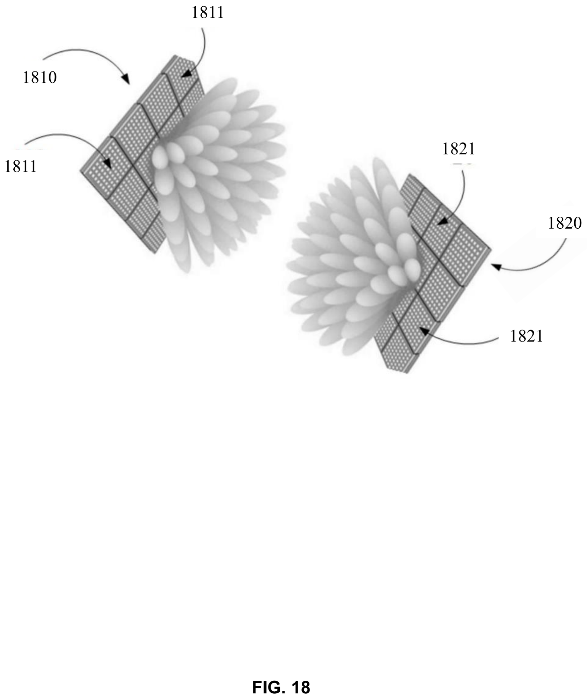

FIG. 18 is a schematic diagram of phased-array elements according to some embodiments of the present disclosure.

As shown in FIG. 8, the phased-array elements include a phased-array transmitting element 1810 and a phased-array receiving element 1820.

The phased-array transmitting element 1810 is an array of a plurality of transmitter antennas 1811. By adjusting the phases of the plurality of transmitting antennas 1811 in the phased-array transmitting element 1810, mutual combinations of different transmitting phases may be realized, and the transmission of radio frequency beams from different angles may be realized. The count of the phased-array transmitting elements 1810 may be set according to the actual demand, such as one, two, three, etc.

The phased-array receiving element 1820 is an array of at least one receiving antenna 1821. By adjusting the phases of the plurality of receiving antennas 1821, mutual combinations of different receiving phases are realized, so as to perform reception of the radio frequency beams from different angles. The count of the phased-array receiving antennas 1821 may be set according to the actual demand, such as one, two, three, etc.

In some embodiments of the present disclosure, by setting at least one receiving antenna to receive the radio frequency beams sent by a plurality of transmitting antennas in the phased-array transmitting element, it is possible to transmit a plurality of radio frequency beams at the same time, realizing parallel communication, thereby significantly enhancing the communication capacity and realizing the high-speed transmission of the plurality of radio frequency beams, saving the transmission time of the signal data generated by the interaction between the rays detected by the detector and the tissue of the scanned object in the medical device 110, improving the signal transmission efficiency, and saving the detection time of the medical device 110.

In some embodiments of the present disclosure, the phased-array transmitting element can realize that the radio frequency beams are received by the phased-array receiving antenna when switching rapidly among different angular directions, and is no longer limited to the requirement of relative positions of the phased-array transmitting antenna and the phased-array receiving antenna in the conventional technology, which improves the quality of the signal communication, so that the signals generated by the interaction of the rays contained in the radio frequency beam with the target object may be accurately applied to the reconstruction of the image in the medical device 110, thereby providing reliable and accurate data for medical diagnosis.

FIG. 19 is a flow diagram of a data transmission process of a transmission system of a medical device according to some embodiments of the present disclosure.

In some embodiments, the transmission system 200 further includes a synchronization module 1740 and a beam controller.

The synchronization module 1740 is configured to obtain real-time motion parameters of the rotor 211 and transmit the real-time motion parameters to the beam controller.

In some embodiments, the synchronization module 1740 may be connected to the rotor 211 via a cable or wirelessly to actively acquire the real-time motion parameters of the rotor in real-time and transmit the real-time motion parameters to the beam controller.

In some embodiments, the synchronization module 1740 is connected to a drive motor at the rotor 211. The drive motor drives the rotation of the rotor 211, which in turn allows a rotation angle of the rotor 211 to be calculated by the rotational speed of the drive motor, and thus the position of the rotor 211 is known in real-time.

The rotor 211 may also actively send the real-time motion parameters in real-time to the synchronization module 1740, and the synchronization module 1740 transmits the real-time motion parameters to the beam controller.

In one embodiment, the beam controller may also be integrated in the same unit as the synchronization module 1740.

The beam controller is configured to adjust radio frequency beams emitted from the first phased-array antenna 1710 or the second phased-array antenna 1720 based on the motion parameters of the rotor 211 and a pre-stored mapping relationship between motion parameters and beam parameters, so that the radio frequency beams are received.

In some embodiments, the motion parameters of the rotor 211 include one or more of angular regions, a speed of the rotor 211, and an acceleration of the rotor 211. The first phased-array antenna 1710 is located at the rotor 211. When the medical device 110 operates, the rotation of the rotor 211 drives the first phased-array antenna 1710 to rotate. The motion of the rotor 211 may be known based on the motion parameter of the rotor 211, and thus the rotation of the first phased-array antenna 1710 may be known. Thereby, the beam controller, based on the motion parameters of the rotor 211, can adjust the radio frequency beams in real-time to be received by the second phased-array antenna 1720. Through the beam controller, the radio frequency beams may be adjusted in real-time, so that the radio frequency beam may be transmitted between the first phased-array antenna 1710 and the second phased-array antenna 1720 at different angles, improving the signal communication quality.

In some embodiments, the beam controller may also obtain the communication quality of the radio frequency beams transmitted between the first phased-array antenna 1710 and the second phased-array antenna 1720 in real-time, and adjust the radio frequency beam according to the communication quality and a communication quality threshold so that the radio frequency beams are transmitted by the second phased-array antenna 1720.

In some embodiments, the communication quality of the radio frequency beams may include one or more of a signal strength, a bit error rate, a signal-to-noise ratio, a throughput, and a data transfer rate of the radio frequency beams. The beam controller adjusts the radio frequency beams in real-time on the basis of the communication quality, which can further realize high-quality signal transmission between the first phased-array antenna 1710 and the second phased-array antenna 1720.

In some embodiments, the beam controller may include a calibration module with a processing module.

The calibration module is configured to pre-store the mapping relationship between the motion parameters and the corresponding beam parameters.

The first phased-array antenna 1710 is located at the rotor 211 and has a fixed relative positional relationship with the rotor 211. When the medical device 110 is in operation, the rotor rotates the first phased-array antenna 1710, and therefore, the radio frequency beams transmitted by the first phased-array antenna 1710 and/or the radio frequency beams received by the second phased-array antenna 1720 have a correspondence with the motion parameters of the rotor 211.

The mapping relationship refers to the correspondence between the motion parameters of the rotor 211 and the beam parameters. The beam parameters may include a beam parameter of the radio frequency beams transmitted by the first phased-array antenna 1710 and/or a beam parameter of the radio frequency beams received by the second phased-array antenna 1720.

In some embodiments, the first phased-array antenna 1710 and the second phased-array antenna 1720 may be calibrated so that the radio frequency beams may be transmitted or received between the first phased-array antenna 1710 and the second phased-array antenna 1720, and the mapping relationship of the motion parameters and the corresponding beam parameters may be determined in the calibration and pre-stored in the calibration module.

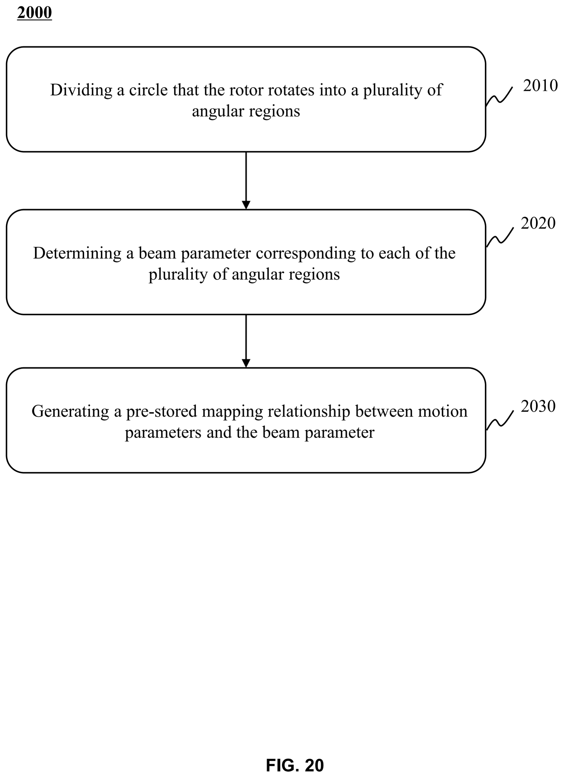

Further description of the mapping relationship of the motion parameters and the corresponding beam parameters and their determination may be found in FIG. 20 of this disclosure and its associated description.

The processing module is configured to determine the beam parameters based on the mapping relationship invoked by the motion parameters at the rotor 211 to adjust pointing of the radio frequency beams.

In some embodiments, the processing module is connected to the first phased-array antenna 1710 to determine the beam parameters of the radio frequency beams emitted by the first phased-array antenna 1710 based on the motion parameter of the rotor 211 and the mapping relationship, and to adjust the pointing of the radio frequency beams emitted by the first phased-array antenna 1710.

In one embodiment, the processing module is connected to the second phased-array antenna 1720 to determine, based on the motion parameter of the rotor 211 and the mapping relationship, the beam parameter of the radio frequency beams received by the second phased-array antenna 1720, to adjust the receiving pointing of the second phased-array antenna 1720.

In one embodiment, the processing module is connected to the first phased-array antenna 1710 and the second phased-array antenna 1720, respectively. The processing module determines, based on the motion parameter of the rotor 211 and the mapping relationship, the beam parameters of the radio frequency beams transmitted by the first phased-array antenna 1710 and the beam parameters of the radio frequency beams received by the second phased-array antenna 1720, to adjust the pointing of the radio frequency beams transmitted by the first phased-array antenna 1710 and the receiving pointing of the second phased-array antenna 1720. By simultaneously adjusting the beam parameters of the radio frequency beams transmitted by the first phased-array transmitting antenna 1710 and the beam parameters of the radio frequency beams received by the second phased-array antenna 1720, it is possible to make a phased-array transmit phase of the first phased-array antenna 1710 and a phased-array receiving phase of the second phased-array antenna 1720 to be adjusted simultaneously in real-time, so that the first phased-array antenna 1710 and the second phased-array antenna 1720 may be adjusted to follow each other, which are no longer limited by the positional limitation of the first phased-array antenna 1710, and the positional limitation of the second phased-array antenna 1720.

The pre-stored mapping relationship between the motion parameters and corresponding beam parameters in the calibration module characterizes a calibration relationship between the first phased-array antenna 1710 and the second phased-array antenna 1720, and can ensure that in the rotation of the first phased-array antenna 1710 following with the rotor 211, high-quality wireless communication between the first phased-array antenna 1710and the second phased-array antenna 1720 is conducted in real-time.

The processing module invokes the mapping relationship based on the motion parameters of the rotor 211, and determines the beam parameters of the radio frequency beams transmitted by the first phased-array antenna 1710 and/or the beam parameters of the radio frequency beams received by the second phased-array antenna 1720, so that the radio frequency beams emitted by the transmitting unit 1810 from different angular directions may be received by the second phased-array antenna 1720, thereby improving the quality of the signal communication to enable the signals generated by the interaction of the rays contained in the radio frequency beams with the target object to be accurately applied to the reconstruction of the images in the medical device 110.

In some embodiments, the transmission system 200 further includes a slide rail, and a support frame 1750 is located at the slide rail to enable a position of the second phased-array antenna 1720 to be moved along the slide rail. In turn, using the slide rail, it is possible to make the relative position of the support frame 1750 and the rotor 211 unrestricted, which may be set according to the actual application scenario, so as to conveniently realize the movement and fixation of the position.

As shown in FIG. 19, through the transmission system for the medical device shown in some embodiments of the present disclosure, a process 1900 of data transmission may be performed.

In 1910, radio frequency beams are transmitted via a phased-array transmitting element (e.g., the phased-array transmitting element 1810 or the first phased-array antenna 1710).

In 1920, motion parameters of the rotor 211 are obtained.

In 1930, pointing of the radio frequency beams is adjusted based on the motion parameters.

In 1940, the radio frequency beams are received through a phased-array receiving antenna (e.g., the receiving antenna 1821 or the second phased-array antenna 1720).

The relevant descriptions of operation 1910, operation 1920, operation 1930, and operation 1940 may be referred to the relevant descriptions in other embodiments in this disclosure.

In one embodiment, in 1930, adjusting the pointing of the radio frequency beams based on the motion parameters comprising: obtaining the pre-stored mapping relationship between the motion parameters and the corresponding beam parameters; determining the beam parameters to adjust the pointing of the radio frequency beams based on the motion parameters of the rotor 211 and the mapping relationship. For more relevant descriptions, reference may be made to relevant descriptions in other embodiments in this disclosure.

In one embodiment, the at least one first phased-array antenna 1710 transmits data at different frequencies or different time interval. Further relevant descriptions may be found in other embodiments in this disclosure.

In some embodiments, the transmission system 200 may also have a closed-loop control module. The closed-loop control module can monitor, in real-time, a signal quality of the signals received by the second phased-array antenna 1720 during the operation of the medical device 110. In response to determining that the signal quality does not satisfy a preset signal quality condition, the closed-loop control module updates, in real-time, the pre-stored mapping relationship, so that the processing module can adjust the pointing of the radio frequency beams according to the updated mapping relationship. Thus, the signal quality of the signals received by the second phased-array antenna 1720 meets the preset signal quality condition.

FIG. 20 is a flow diagram for determining a pre-stored mapping relationship between motion parameters and beam parameters according to some embodiments of the present disclosure. As shown in FIG. 20, the process 2000 includes:

In 2010, a circle that the rotor rotates is divided into a plurality of angular regions.

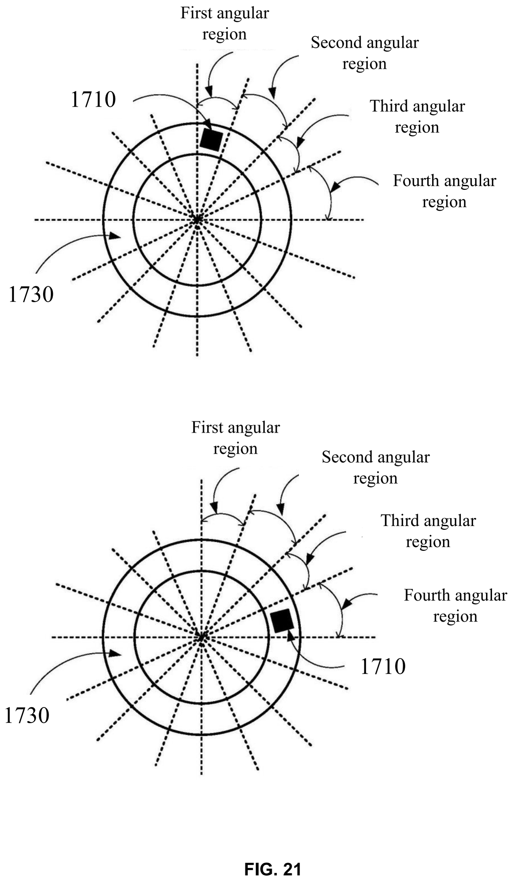

FIG. 21 is a schematic diagram of angular regions according to some embodiments of the present disclosure.

An angular region of the plurality of angular regions refers to a specific angular range of a circular region formed by the rotation of the first phased-array antenna 1710 located at the rotor 211. As shown in FIG.21, the plurality of angular regions include a first angular region, a second angular region, a third angular region, a fourth angular region, etc.

Each angular region corresponds to one rotation angular range of the rotor 211, and also corresponds to one rotation angular range of the first phased-array antenna 1710. When the medical device 110 operates, the rotation of the rotor 211 drives the rotation of the first phased-array antenna 1710, causing the position of the first phased-array antenna 1710 to change. Thereby, the angle of rotation of the rotor 211 may be known by the angular regions, and the angle of rotation of the first phased-array antenna 1710 can also be known. The angular regions are different, and the beam parameters of the radio frequency beams emitted by the first phased-array antenna 1710 are different.

Exemplarily, the circular region formed by the rotation of the rotor 211 may be divided into a plurality of angular regions. For example, the angle through which the rotor 211 rotates in one complete circle is 360°. There is an angular region for every interval of 30°. Thus, a count of the plurality of angular regions is 12 (360°/30=12). As another example, a count of the plurality of angular regions is 16 or more according to the actual application scenarios. As another example, the plurality of angular regions are evenly divided or unevenly divided.

In 2020, a beam parameter corresponding to each of the plurality of angular regions is determined.

The beam parameter is a set of indicators that quantitatively characterize the spatial distribution of the radiation beam of the phased-array antenna. For example, the beam parameter includes a beam pointing angle, a main flap width, a sub-flap level, a zero depth, and a scanning range, etc.

The first phased-array antenna 1710 (e.g., the phased-array transmitting element 1810) is located at the rotor 211. When the real-time rotational position of the rotor 211 is determined to be the first angular region or the fourth angular region, signal scanning and positioning each antenna or element in the first phased-array antenna 1710 (e.g., the phased-array transmitting element 1810) and the second phased-array antenna 1720 (e.g., the phased-array receiving antenna 1821) to determine signal transmission directions. The beam parameters corresponding to the transmission direction of the communication signal that meets error rate requirements are determined, thereby obtaining the calibrated beam parameters (e.g., transmission phase information and reception phase information). The transmission phase information is configured to control the phase of the first phased array antenna 1710. The reception phase information is configured to control the phase of the second phased array antenna 1720. Therefore, when the rotation angle area position in the motion parameters is different, it may correspond to different beam parameters.

In some embodiments, the beam parameters corresponding to the first phased-array antenna 1710 at different angles of rotation may be obtained in a plurality of ways. For example, by performing theoretical calculations based on the array factor theory, simulating the actual operation of the first phased-array antenna 1710 by electromagnetic simulation, or calibrating the first phased-array antenna 1710 by a pre-conducted experimental real measurement at the corresponding beam wave parameters at different rotation angles, etc.

In 2030, the pre-stored mapping relationship between the motion parameters and the beam parameter is generated.

The motion parameters are data related to a motion state of the rotor 211 and/or the first phased-array antenna 1710.

In some embodiments, the motion parameter may include at least one of an angular region, a velocity, an acceleration, or a movement parameter.

In some embodiments, the mapping relationship may include a mapping relationship between the angular regions and the beam parameters. At this point, the motion parameter includes the angular regions where the first phased-array antenna is located. The mapping relationship between the angular regions and the beam parameter may be determined based on the beam parameter corresponding to each of the plurality of angular regions.

In some embodiments, the mapping relationship may further include a mapping relationship between a speed of the rotor 211 and the beam parameters. At this point, the motion parameter may also include the speed of the rotor 211.

The speed of the rotor 211 characterizes the motion of the rotor 211 and the rotation of the first phased-array antenna 1710. By means of the calibration module, the mapping relationship between the motion parameters of the rotor 211 and the corresponding beam parameters during the calibration process is established on the basis of the communication signals that satisfy the error rate requirement. The mapping relationship between the speed of the rotor 211 and the corresponding beam parameters is established during the rotation of the rotor 211. Thus, different speeds of the rotor 211 in the motion parameters correspond to different beam parameters.

In some embodiments, the mapping relationship may also include a mapping relationship between an acceleration at the rotor 211 and the beam parameters. At this point, the motion parameters may also include the acceleration of the rotor 211.

When the acceleration at the rotor 211 changes, the corresponding beam parameters change. Thus, the mapping relationship between the acceleration of the rotor 211 and the corresponding beam parameter is established during the rotation of the rotor 211.

The processing module, based on the motion parameters of the rotor 211, may determine the beam parameters based on the mapping relationship. According to the beam parameters, the pointing of the radio frequency beams may be adjusted to enable high quality transmission of the radio frequency beams between the first phased-array antenna 1710 (e.g., the phased-array transmitting element 1810) and the second phased array antenna 1720 (e.g., the phased-array receiving antenna 1821).

In some embodiments, the mapping relationship between the moment parameters and the beam parameters is pre-stored in the calibration module. The processing module determines the beam parameters based on the mapping relationship that may be called at the current moment to adjust the pointing of the radio frequency beams. The moment at which the rotor begins to rotate is taken as an initial moment, and the mapping relationship between the moment parameters and the beam parameters is established. The processing module, based on the current moment, determines the corresponding beam parameters in the mapping relationship to adjust the beam parameters of the radio frequency beams transmitted by the phased-array transmitting element 1810 or/and the beam parameters of the radio frequency beams received by the phased-array receiving antenna 1821.

In some embodiments, the mapping relationship between the pre-stored motion parameters and the corresponding beam parameters further includes moment parameters. The inclusion of the moment parameter in the calibration process between the phased-array transmitting element 1810 and the phased-array receiving antenna 1821 allows for the moment of the beginning of the rotation of the rotor 211 to be used as the initial moment to establish the mapping relationship between the moment parameters, the motion parameter, and the beam parameters.

In some embodiments, the mapping relationship between the motion parameters, the moment parameters, and the beam parameters is pre-stored in the calibration module.

In some embodiments, the mapping relationship may be in the form of a database, and the processing module directly accesses the database of the mapping relationship to realize the adjustment of the beam parameters of the radio frequency beams emitted by the phased-array transmitting element 1810 or/and the beam parameters of the radio frequency beams received by the receiving antenna 1821.

In some embodiments, the mapping relationship may be in the form of a learning model (e.g., a machine learning model), which serves as a carrier of the mapping relationship and continuously learns the mapping relationship between the motion parameters and the beam parameters. The processing module uses the mapping relationship established by the learning model to realize the adjustment of the beam parameters of the radio frequency beams emitted by the phased-array transmitting element 1810 or/and the beam parameters of the radio frequency beams received by the phased-array receiving antenna 1821.

In some embodiments, by establishing the mapping relationship between the motion parameters and the beam parameters, it is possible to enable the processing module to determine the corresponding beam parameters in the mapping relationship according to the current moment and the motion parameters, to adjust the beam parameters of the radio frequency beams emitted by the phased-array transmitting elements or/and the beam parameters of the radio frequency beams received by the phased-array receiving antenna. By incorporating the moment parameters and establishing the mapping relationship among the motion parameters, the moment parameters, and the beam parameters, the processing module can invoke the corresponding beam parameters in the mapping relationship according to the current moment to more accurately adjust the beam parameters of the radio frequency beams transmitted by the phased-array transmitting element or/and the beam parameters of the radio frequency beams received by the phased-array receiving antenna.

Some embodiments of the present disclosure further provide a medical device comprising the transmission system 200 according to some embodiments, a detailed description of which may be found in the relevant preceding description.

The basic concepts have been described above, and it is apparent to a person skilled in the art that the above detailed disclosure serves only as an example and does not constitute a limitation of this disclosure. While not expressly stated herein, various modifications, improvements, and amendments may be made to this disclosure by those skilled in the art. Those types of modifications, improvements, and amendments are suggested in this disclosure, so those types of modifications, improvements, and amendments remain within the spirit and scope of the exemplary embodiments of this disclosure.

Also, this disclosure uses specific words to describe the embodiments of this disclosure, such as "an embodiment", "an embodiment", and/or "some embodiment" means a feature, structure, or characteristic associated with at least one embodiment of the present disclosure. Accordingly, it should be emphasized and noted that "one embodiment" or "one embodiment" referred to two or more times in different locations in this disclosure or "a count of embodiments" means a feature, structure, or characteristic related to at least one embodiment of this disclosure. "one embodiment" or "an alternative embodiment" in different places in this disclosure do not necessarily refer to the same embodiment. In addition, certain features, structures, or characteristics in one or more embodiments of the present disclosure may be suitably combined.

**In** addition, the order of processing elements and sequences, the use of numerical letters, or the use of other names described herein are not intended to qualify the order of the processes and methods of this disclosure, unless expressly stated in the claims. While some embodiments of the invention that are currently considered useful are discussed in the foregoing disclosure by way of various examples, it should be appreciated that such details serve only illustrative purposes and that additional claims are not limited to the disclosed embodiments, rather, the claims are intended to cover all amendments and equivalent combinations that are consistent with the substance and scope of the embodiments of this disclosure. For example, although the implementation of various components described above may be embodied in a hardware device, it may also be implemented as a software only solution, e.g., an installation on an existing server or mobile device.

Similarly, it should be noted that in order to simplify the presentation of the disclosure of this disclosure, and thereby aid in the understanding of one or more embodiments of the invention, the foregoing descriptions of embodiments of this disclosure sometimes combine a plurality of features into a single embodiment, accompanying drawings, or the description thereof. However, this method of disclosure does not imply that the objects of the present disclosure require more features than those mentioned in the claims. Rather, claimed subject matter may lie in less than all features of a single foregoing disclosed embodiment.

Numbers describing the count of compositions, attributes are used in some embodiments, and it should be understood that such numbers used in the description of embodiments, in some examples, use the modifiers "about ", "approximately", or "generally" is used in some examples. Unless otherwise noted, the terms "about," "approximate," or "approximately" indicate that a ±20% variation in the stated number is allowed. Correspondingly, in some embodiments, the numerical parameters used in the disclosure and claims are approximations, which can change depending on the desired characteristics of the individual embodiment. In some embodiments, the numerical parameters should take into account the specified count of valid digits and use a general digit retention method. While the numerical domains and parameters used to confirm the breadth of their ranges in some embodiments of this disclosure are approximations, in specific embodiments such values are set to be as precise as possible within the feasible range.

For each of the patents, patent applications, patent application disclosures, and other materials cited in this disclosure, such as articles, books, disclosure sheets, publications, documents, or the like, the entire contents of which are hereby incorporated herein by reference. Application history documents that are inconsistent with or conflict with the contents of this disclosure are excluded, as are documents (currently or hereafter appended to this disclosure) that limit the broadest scope of the claims of this disclosure. It is to be noted that to the extent that there is an inconsistency or conflict between the descriptions, definitions, and/or use of terms in the materials appurtenant to this disclosure and those set forth in this disclosure, the descriptions, definitions and/or use of terms in this disclosure shall prevail. use shall prevail.

Finally, it should be understood that the embodiments described in this disclosure are used only to illustrate the principles of the embodiments of this disclosure. Other deformations may also fall within the scope of this disclosure. As such, alternative configurations of embodiments of the present disclosure may be viewed as consistent with the teachings of the present disclosure as an example, not as a limitation. Correspondingly, the embodiments of the present disclosure are not limited to the embodiments expressly presented and described herein.

## Claims

1. A transmission system of a medical device, comprising a first transceiver on a rotor and a second transceiver on a stator, wherein
the first transceiver on the rotor is configured to transmit scanning data to and receive control signals from the second transceiver on the stator;
the second transceiver on the stator is configured to transmit the control signals to and receive the scanning data from the first transceiver on the rotor; and
a bidirectional transmission between the first transceiver and the second transceiver is wireless.

2. The transmission system of claim 1, wherein the bidirectional transmission between the first transceiver and the second transceiver is a transmission via wave-based signals.

3. The transmission system of claim 1 or claim 2, wherein the bidirectional transmission between the first transceiver and the second transceiver is a transmission via optical waves, and the transmission system further comprises a beam expander, wherein
the first transceiver or the second transceiver is configured to emit a first optical beam carrying data;
the beam expander is configured to divide the first optical beam into a plurality of second optical beams, each of the plurality of second optical beams records at least part same data with the first optical beam; and
the second transceiver or the first transceiver is configured to receive at least part of the plurality of second optical beams.

4. The transmission system of claim 3, wherein the beam expander includes a wavelength division demultiplexer and a filter, the wavelength division demultiplexer is configured to divide the first optical beam into the plurality of second optical beams of a plurality of wavelengths, and the filter is configured to filter the plurality of second optical beams.

5. The transmission system of claim 3 or claim 4, further comprising an angle determination module, wherein the angle determination module is configured to:
determine an angle covered by an expanded optical spot; and
determine, based on the angle, a first transceiver count or a second transceiver count, and an interval angle between two adjacent first transceivers or an interval angle between two adjacent second transceivers.

6. The transmission system of claim 5, wherein
the transmission system includes a plurality of first transceivers and a second transceiver, the plurality of first transceivers are arranged at interval along a circumference of the rotor based on the interval angle; or
the transmission system includes a first transceiver and a plurality of second transceivers, the plurality of second transceivers are arranged outside a circumference of the rotor at interval based on the interval angle.

7. The transmission system of any one of claims 1-6, wherein the transmission system comprises a plurality of first transceivers or a plurality of second transceivers, and the plurality of first transceivers or the plurality of second transceivers are arranged along a circumference of the rotor and/or the stator.

8. The transmission system of claim 1, wherein the bidirectional transmission between the first transceiver and the second transceiver is a transmission via optical waves, wherein
the first transceiver is located on a circumference of the rotor, the stator is located on a rotational axis of the rotor;
the transmission system further includes an optical path adjustment module between the first transceiver and the second transceiver; and
the optical path adjustment module is configured to adjust an optical direction of an optical beam emitted by the first transceiver or the second transceiver.

9. The transmission system of claim 8, wherein the optical path adjustment module includes at least one adjustment unit located adjacent to the first transceiver of the second transceiver that serves as a receiver.

10. The transmission system of claim 8 or claim 9, wherein the optical path adjustment module includes a first adjustment unit located adjacent to the first transceiver of the second transceiver that serves as a receiver and a second adjustment unit located adjacent to the first transceiver of the second transceiver that serves as a transmitter.

11. The transmission system of any one of claims 8-10, further comprising
a controller configured to control the optical path adjustment module, and
a power detector configured to detect a power of an optical beam received by the first transceiver or the second transceiver, and transmit the power to the controller, wherein the controller controls the optical path adjustment module based on the power; and/or
a vibration detector configured to detect vibration information of the rotor and transmit the vibration information to the controller, wherein the controller controls the optical path adjustment module based on the vibration information.

12. The transmission system of claim 1, wherein the bidirectional transmission between the first transceiver and the second transceiver is the transmission via electromagnetic waves, wherein
the first transceiver is located on a circumference of the rotor;
the first transceiver includes a first phased-array antenna; and
the second transceiver includes a second phased-array antenna.

13. The transmission system of claim 12, wherein the second phased-array antenna is located within a coverage area of radio frequency beams emitted from the first phased-array antenna.

14. The transmission system of claim 12 or claim 13, further comprising:
a synchronization module configured to obtain real-time motion parameters of the rotor;
a beam controller configured to adjust the radio frequency beams emitted from the first phased-array antenna or the second phased-array antenna based on the motion parameters of the rotor and a pre-stored mapping relationship between motion parameters and beam parameters.

15. A medical device comprising a transmission system according to any one of claims 1-14.
